# EUROPEAN PATENT SPECIFICATION

(11) **EP 3 938 077 B1**
(45) Date of publication and mention of the grant of the patent: **10.09.2025**
(21) Application number: 20770261.4
(22) Date of filing: 12.03.2020
(51) Int. Cl.: B01F 29/30, B01F 35/71, B01F 35/33, B01F 33/30, B01F 33/25, G01N 35/00, G01N 33/58, G01N 33/53, G01F 5/00, G01F 1/40, G01F 11/28, G01F 1/20, B01L 3/00, B01D 46/44

(54) **POINT OF CARE CONCENTRATION ANALYZER AND METHOD OF MIXING**
KONZENTRATIONSANALYSATOR FÜR DEN EINSATZ AM VERSORGUNGSORT UND MISCHVERFAHREN
ANALYSEUR DE CONCENTRATION DE POINT D'INTERVENTION ET PROCÉDÉ DE MÉLANGE

(30) Priority: 12.03.2019 US 201962817433 P
(43) Date of publication of application: 19.01.2022
(73) Proprietor: Novilux, LLC, Bainbridge Island, WA 98110 (US)
(72) Inventor: ORTYN, William, E., Bainbridge Island, WA 98110 (US); NOLAND, Brian, W., Livermore, CA 94550 (US); BISHOP, Jeffrey, J., Dublin, CA 94568 (US); LIVINGSTON, Richard, Webster Groves, MO 63119 (US); ONORATO, Robert, Alameda, CA 94501 (US); GILBERT, Michele, Albany, CA 94706 (US); WANG, Danni, Albany, CA 94501 (US); KYGER, Erich, Antioch, CA 94531 (US); JOLLY, John, David, Poulsbo, WA 98370 (US); MONSALVE, Luis, H., Bainbridge Island, WA 98110 (US)
(74) Representative: Mewburn Ellis LLP
(86) International application number: PCT/US2020/022399
(87) International publication number: WO 2020/186062

(56) References cited:
- EP-A2- 1 894 617
- WO-A1-2008/103116
- WO-A1-97/08556
- JP-B1- 6 434 114
- US-A- 5 912 134
- US-A1- 2005 029 440
- US-A1- 2010 196 908
- US-A1- 2012 214 224
- US-A1- 2015 064 693
- US-A1- 2015 346 097
- US-A1- 2019 011 470
- US-A1- 2019 054 468
- US-A1- 2019 162 670

## Description

### FIELD

This invention generally relates to the automated sample processing, measurement and analysis of samples in order to isolate, label, detect and determine the amount of specific target analytes that may be present in very low concentrations.

### BACKGROUND

Numerous studies and advances in the understanding of the underlying causes and the progression of disease have shown that detection of infectious agents or the detection of an impairment at an early stage coupled with appropriate treatment substantially improves clinical outcomes. Many conditions that once required the use of expensive symptomatic measurements like anatomical imaging, which require trained specialist to administer and interpret, can now be diagnosed at the cellular and molecular level via the presence and / or concentration of specific biomarkers. These biomarkers include up- or down-regulated proteins, nucleic acids or other molecules that are highly specific to a disease condition or infection.

It is often desirable to diagnose certain conditions at the point-of-care where timing and administration of the correct treatment is critical for patient outcome. This is especially true in the acute care settings of trauma centers where patients may have experienced an acute myocardial infarction (AMI), acutely decompensated heart failure, pulmonary embolism, sepsis, or other conditions requiring a timely response. In non-acute settings a quick turnaround time is also desirable especially in the case of highly infectious diseases like *C. difficile* infection where a quarantine might be required. However, even in the doctor's office or retail clinic it is highly beneficial to determine if a condition is viral or bacterial prior to administering antibiotics.

With some disease conditions the concentration of the biomarker or analyte of interest is relatively high and simple low-cost lateral flow devices may be employed for sample processing and readout. These devices and the consumable components that interact with the sample are very low cost and can be used quickly with relatively little or no training at the point-of-care. However, lateral flow type tests also tend to suffer from poor precision making quantitative measurements of marginal quality even if an objective reader system is used to measure the strips. Also, depending on the stage of a disease or infection the concentration of the target analyte is often too low to detect with lateral flow in the blood, urine, saliva or other sample types.

In these cases the sample processing and readout is more complex. It often requires precise metering to assess concentration, high efficiency to avoid loss of the target analyte and centrifugal separation as a primary step in the purification process. Beyond centrifugation, additional purification steps typically include incubation with reagents containing binding partners or molecules with complimentary sequences or structures to bind to the target analyte biomarker. These binding partners may be substrates such as micro- or nanoparticles with complimentary molecules on their surfaces or molecules conjugated to transduction labels, or both. Once binding occurs additional process steps must then be taken to wash and further isolate the target analyte to suspend it in pristine buffer solution or lay it down on a clean surface prior to measurement. To perform these processing steps, multiple devices including centrifuges, mixers, incubators, precision pipettors, and thermal cyclers are used wherein the sample is often transferred and metered between the processing steps with multiple disposable tips, tubes, plates and other sample containers etc. Once the processing is complete highly sensitive and highly precise instruments are used to measure the processed sample to determine the presence and or abundance of the target analyte.

While the analysis of low concentration biomarkers may take several forms, in general the processing and measurement of the sample has the following principal characteristics:
1. A separation step to perform a first isolation of the target analyte from other sample components
2. Introduction of binding partners and reagents
3. Mixing and incubation to label and bind target analytes to a substrate
4. Introduction of buffers and steps to wash away unbound labels and other contaminates
5. Sterile containers for precise metering and sample containment during processing
6. An efficient and precise means of sample transfer
7. A means of measurement providing high sensitivity and precision to determine the presence and abundance of the target analyte.

At present the processing and measurement of low concentration biomarkers must be done by trained staff or with the use of highly specialized equipment in centralized locations. Consequently, the turn-around time from sample acquisition to result is long, the instrumentation cost is high, and the measurement cannot be done at the point-of-care.

Accordingly, the present inventors have recognized that an improved technique that can address the principal characteristics listed above for low concentration biomarker processing and measurement is desired. The technique should be suitable for the point-of-care environment with minimal consumables, precise metering, fast turnaround time, and with sensitivity that overcomes the limitations of the prior art.

U.S. Patent No. 8,264,684, and U.S. Patent Application Publication No. 2016/0178520, describe previous systems that achieved extremely sensitive detection. The disclosure provides further development in this field.

WO 2008/103116 A1 describes a method of mixing aliquots in a microchannel structure. EP 1 894 617 A2 describes a method of mixing at least two kinds of fluids in centrifugal micro-fluid treating substrate, in accordance with the preamble of claim 1. US 5,912,134 describes a disposable cartridge and method for an assay of a biological sample. WO 97/08556 describes an analytical rotor and method for detecting analytes in liquid samples. JP 6434114 (equivalent to US 2019/0162670 Al) describes a measurement method and measurement device. US 2019/0011470 A1 describes a sample analysis substrate, sample analysis device, sample analysis system, and program for sample analysis. US 2019/0054468 A1 describes devices, systems and methods for sample detection.

### SUMMARY

In a first aspect, the present invention provides a method of mixing a liquid in a cartridge (150) in the form of a flat disc, the method comprising: introducing a liquid to a mixing chamber (175) of the cartridge (150) through a channel that extends radially inward from the mixing chamber, the mixing chamber including a mixing ball (176) therein; rotating the cartridge (150) in a first circumferential direction so as to urge the liquid radially outward and retain the liquid in the mixing chamber (175); and while continuing to rotate the cartridge (150) in the first circumferential direction, intermittently accelerating and decelerating the rotation of the cartridge (150) so as to move the mixing ball (176) back-and-forth recursively through the mixing chamber (150).

In some embodiments, the mixing chamber (175) is provided with lyophilized reagents prior to the introduction of the liquid, and wherein mixing resulting from the movement of the mixing ball (176) through the mixing chamber (175) releases gas from the lyophilized reagents into the liquid. In some embodiments, the released gas moves radially inward and out of the mixing chamber (175).

In some embodiments, the mixing ball (176) is non-magnetic.

In some embodiments, a rotational speed of the cartridge (150) is maintained in a range of 200 to 500 rpm while intermittently accelerating and decelerating, corresponding to a mix frequency of 5Hz.

In some embodiments, the mixing chamber (175) is provided with paramagnetic capture beads (177) prior to introduction of the liquid.

In some embodiments, the channel extending radially inward from the mixing chamber (175) includes an expanded cross-sectional area that forms a capillary break (178).

In some embodiments, the cartridge (150) includes a second channel extending radially inward from the mixing chamber (175), coupling the mixing chamber with a wash chamber (181).

### BRIEF DESCRIPTION OF THE DRAWINGS

The accompanying drawings are included to provide a further understanding of the methods and devices of the disclosure, and are incorporated in and constitute a part of this specification. The drawings are not necessarily to scale, and sizes of various elements may be distorted for clarity. The drawings together with the description serve to explain the principles and operation of the disclosure.
FIG. 1 is a schematic perspective front view of a high sensitivity analyzer as described herein;
FIG. 2 is a schematic perspective rear view of the analyzer of FIG. 1;
FIG. 3 is a schematic perspective view of an optical system used in the analyzer of FIG. 1;
FIG. 4 is a schematic perspective view of a portion of the analyzer of FIG. 1 including a centrifuge;
FIG. 5 is a schematic perspective top view of a portion of the analyzer of FIG. 1 including a manifold;
FIG. 6 is a schematic perspective bottom view of the manifold of FIG. 5;
FIG. 7 is a schematic side view of a portion of the analyzer of FIG. 1 including an objective;
FIG. 8 is a schematic depiction of a fluid system of the analyzer of FIG. 1
FIG. 9 is a schematic top view of a cartridge as described herein;
FIG. 10 is a schematic top view of another cartridge in accordance with a first instance of a method described herein;
FIG. 11 is a schematic top view of the cartridge of FIG. 10 at a second instance of a method described herein;
FIG. 12 is a schematic top view of the cartridge of FIG. 10 at a third instance of a method described herein;
FIG. 13 is a schematic top view of the cartridge of FIG. 10 at a fourth instance of a method described herein;
FIG. 14 is a schematic top view of the cartridge of FIG. 10 at a fifth instance of a method described herein;
FIG. 15 is a schematic top view of the cartridge of FIG. 10 at a sixth instance of a method described herein;
FIG. 16 is a schematic top view of the cartridge of FIG. 10 at a seventh instance of a method described herein;
FIG. 17 is a schematic top view of the cartridge of FIG. 10 at an eight instance of a method described herein;
FIG. 18 is a schematic top view of the cartridge of FIG. 10 at a ninth instance of a method described herein;
FIG. 19 is a schematic top view of a cartridge as described herein;
FIG. 20 is a schematic side view of a portion of the analyzer of FIG. 1 including magnetic stages;
FIG. 21 is a schematic side view of various steps in a washing operating utilizing magnets as described herein; and
FIG. 22 is a schematic side view of various steps in another washing operation utilizing magnets as described herein.

### DESCRIPTION

The following detailed description presents an overview of a method and system as described herein. This overview is followed by further descriptions of various examples of methods, systems and apparatuses.

### Overview

### Separation and Metering

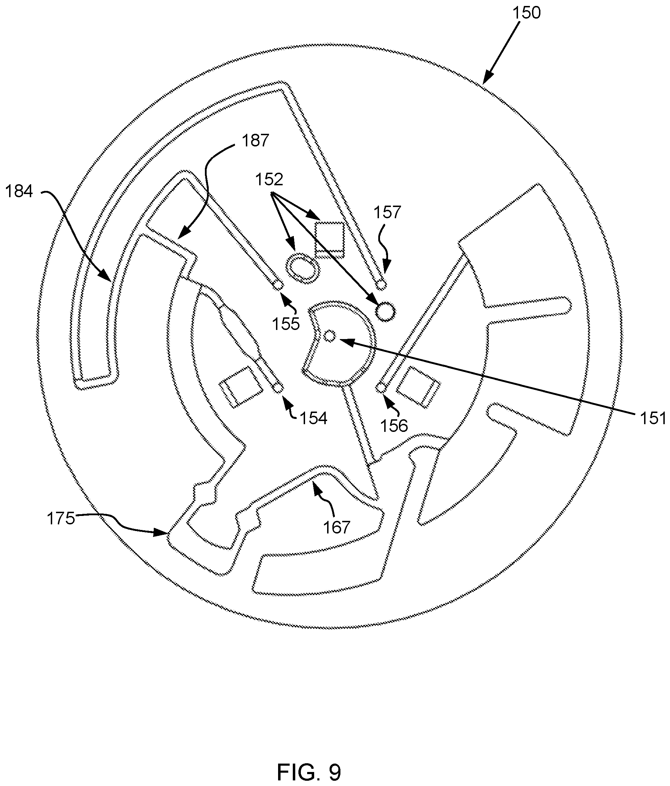

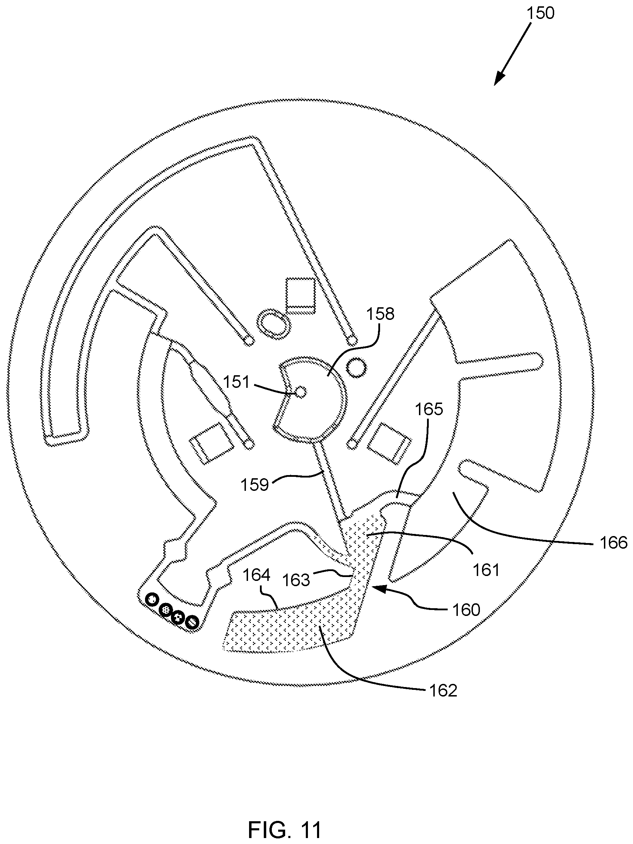

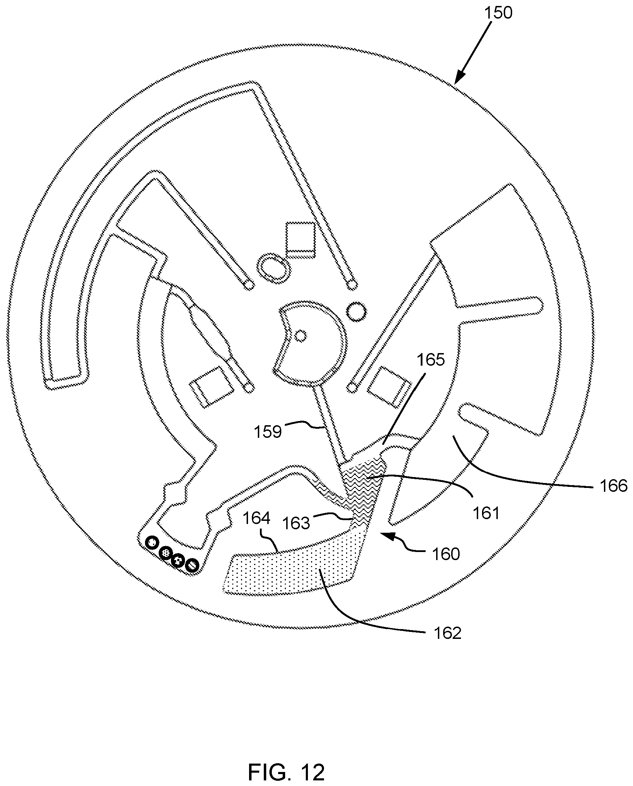

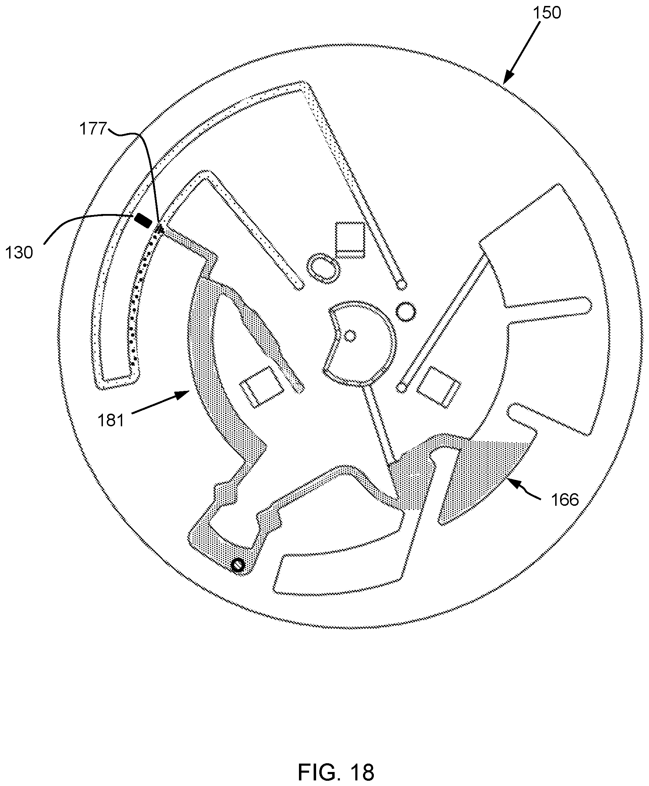

Described herein is a sample processing and analysis system to isolate target analytes and determine their concentration. A cartridge, in the form of a single consumable disc 150, as shown in- FIGS. 9-19 may be used for all sample processing, metering and containment of the resulting processed sample during measurement. The processing involves high speed spinning of the disc to spin down dense elements contained in the original sample. During the initial centrifugation step as shown in FIG. 11, the sample 152 is transferred from a sample chamber 158 to the separation chambers 161, 162 shown in FIG 12. The cartridge 150 is then spun at a higher speed, for example 7000 rpm, to separate dense elements into separation area 162. The resulting supernatant in separation area 161 is then transferred to a mixing chamber 175 containing reagents composed of binding partners. The volumes of the separation chamber 161 and mixing chamber 175 as well as the method of the transfer of the supernatant act to meter the amount of sample used in processing in order to maintain precision. The spin down process and transfer may be imaged by a processing quality control camera and analyzed during processing to ensure proper separation and metering.

### Reagents and Binding Partners

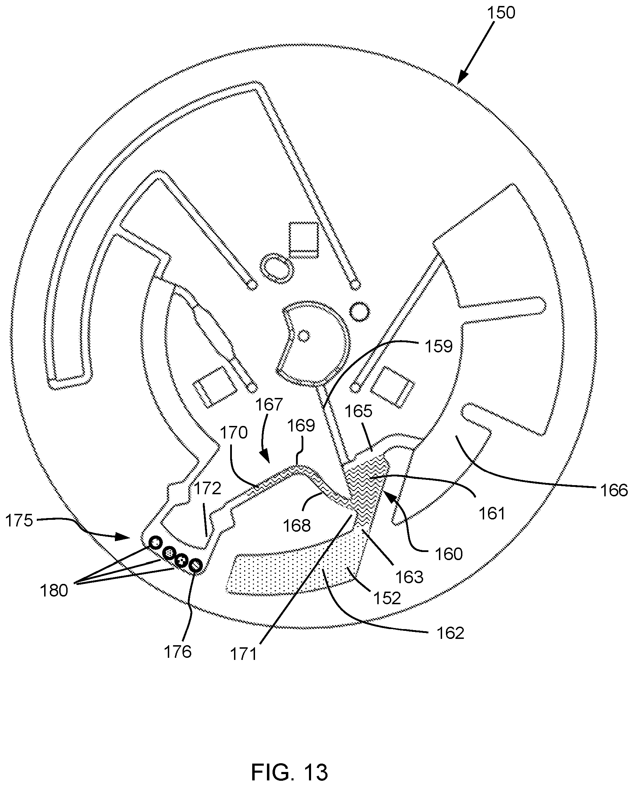

The binding partners include several species which may be in the form of dried reagents or lyophilized pellets 180 shown in FIG. 13, and may be stored in the mixing chamber. The first species are paramagnetic bead substrates that are functionalized with molecules that have binding sites specific to the target analyte. A second species of binding partner includes fluorescent labels conjugated to molecules with binding sites specific to a separate and distinct portion of the target analyte. Another component in the reagents may include a control analyte comprised of an engineered protein or other molecule that would not be found in a patient sample containing the target analyte. In connection with the control analyte is another set of paramagnetic capture beads and labels that are specific to binding sites on the control analyte in the same manner that the first set of paramagnetic capture beads and labels are specific to the target analyte. The control will undergo the same process and measurement as the target analyte. However, the amount of the control, unlike the target analyte, is precisely known before the process. Therefore, when the control is measured, since the concentration is known a priori, it can serve as a monitor of the efficacy of the sample processing and a means for normalizing imprecision in the process.

### Mixing and Incubation

Once the supernatant and reagents are in the mixing chamber, a mixing process will occur in which the disc 150 will spin slowly, but at a variable rpm. Specifically, the disc will accelerate and decelerate rotationally at a controlled rate to execute a preferred motion profile during the spin. As shown in FIG 13 a mixing ball 176 of higher density than the sample, such as brass or glass, is incorporated in the mixing chamber. Beneficially, the acceleration and deceleration of the cartridge will induce the ball to move through the supernatant to facilitate dissolving and disbursement of the dried reagents and ensure a homogeneous mixture of all reagents and the target analyte. The mixing chamber geometry may be configured along a substantially constant radius from the center of rotation. Moreover, this chamber may contain various features to further facilitate mixing and incubation and to ensure the mixture stays in the mixing chamber during the mixing process. The result of the mixing process will promote a homogeneous suspension to facilitate binding of the target analyte to the paramagnetic capture beads and labeled molecules. To ensure precision, the mixing process can be carried out for a controlled amount of time.

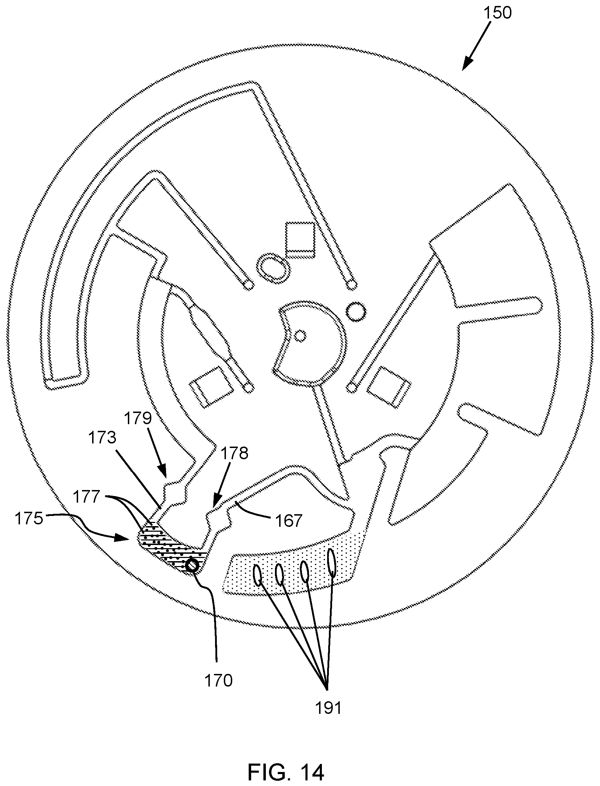

The cartridge 150 also includes a wash chamber 181 that is coupled to the mixing chamber through a channel 173. While the supernatant may otherwise be free to flow into the wash chamber via the channel 173, the wash chamber is advantageously positioned at a smaller radius from the center of rotation such that centrifugal force keeps the supernatant and reagents in the mixing chamber. Capillary breaks in the form of flared channel widths 178, 179 as shown in FIG 14 may be present to further prevent capillary action from pulling the supernatant and mixed suspension from the mixing chamber into the wash chamber. The capillary breaks 178, 179 can also serve to store supernatant which will displace air captured in lyophilized pellets during manufacture of the pellets.

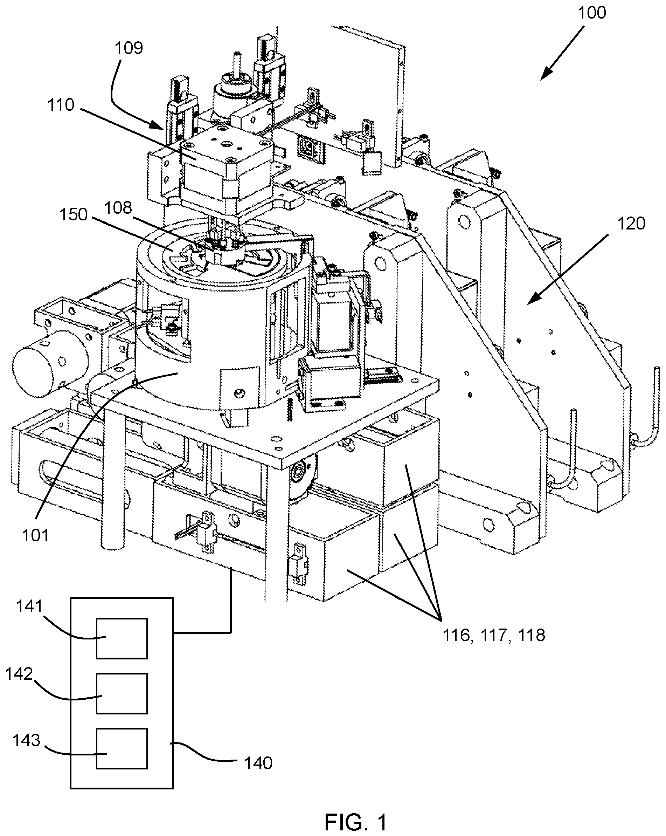

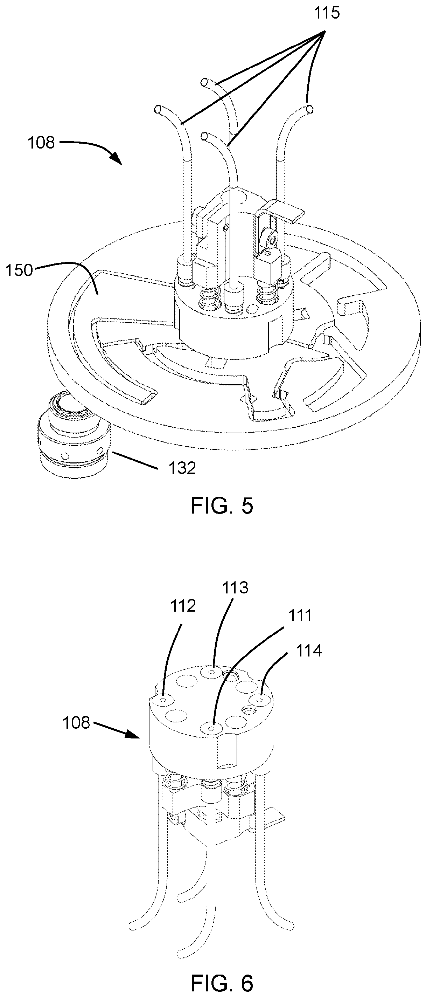

Once the mix and incubation processes are complete, the cartridge 150 may be rotated into alignment with a manifold 108 (FIG. 5) having four ports 111, 112, 113, 114 and associated seals that will move to interface with the cartridge. The manifold may be coupled to a motor 110 for precision rotary movement and the manifold may also be held on a suspension system to ensure contact and sealing of the ports 111, 112, 113, 114 on the cartridge. The manifold may also be on a moveable arm 109 as shown in FIG 1 to lower and raise manifold to and from the cartridge. The manifold may also contain features to precisely register it with respect to the cartridge to ensure the various ports line up with sufficient positional accuracy. Once the manifold is registered and in contact with the cartridge, the manifold motor enables precise rotary motion of the cartridge 150 about the axis of the centrifuge motor. At this point the centrifuge motor may be deenergized and its bearings and shaft can serve as a precision rotary stage for the cartridge. The manifold is connected to a series of pumps and valves to enable the introduction of buffers into the cartridge from external reservoirs through precision syringe pumps. The buffers preferentially include a wash buffer, an elution buffer and DI water for cleaning and storage. Likewise, the ports on this cartridge may include a wash buffer entry port 154, a wash buffer draw port 156, an elution buffer entry port 155 and an elution buffer draw port 157 as shown in FIG. 9.

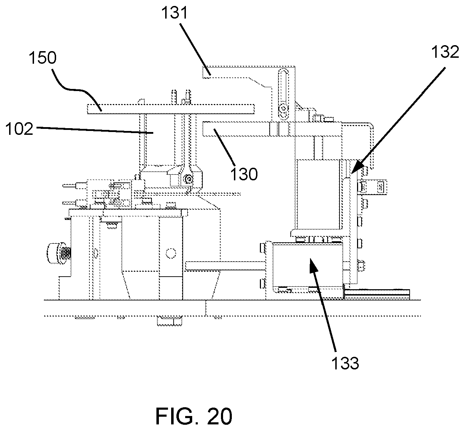

After the manifold 108 is registered on the cartridge 150, one or more magnets are moved into position over the mixing chamber. The magnets are positioned above and below the cartridge on a Z-stage with an axis of motion perpendicular to the flat surface of the cartridge as shown in FIG 20. This enables magnet 130 to move closer to the cartridge 150 increasing its effective pull on the paramagnetic capture beads while the other magnet 131 moves away from the cartridge 150 decreasing its affect. The magnet Z-stage 132 is also coupled to a radial stage 133. The radial stage allows movement of the magnets closer to or away from the axis of rotation of the cartridge. As discussed later, various channels and chambers on the cartridge are arranged radially or circumferentially. The various Z- and radial stages in combination with the manifold motor enable the magnets to be placed at any desired position relative to the chambers and channels contained within the cartridge 150.

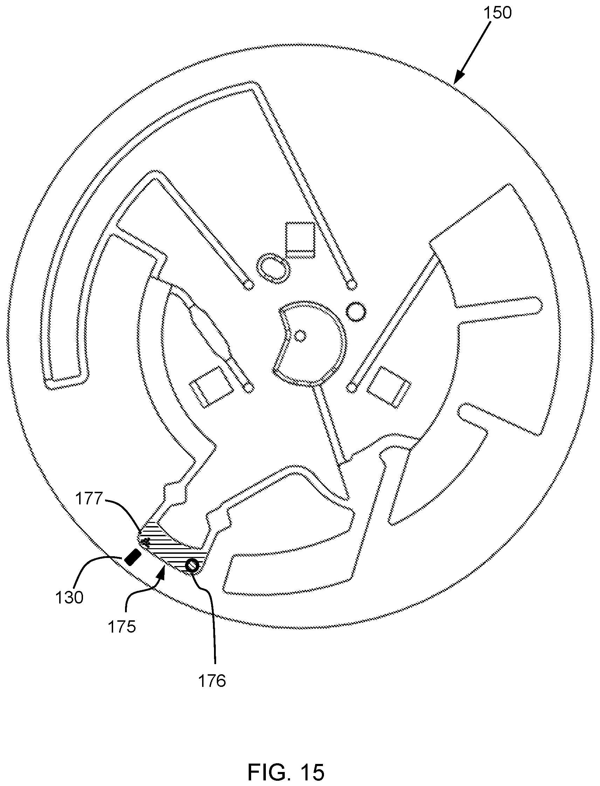

After the magnets are introduced, the magnet Z- and radial stages are controlled along with partial cartridge rotation to execute a predefined sequence of movements to pull all the paramagnetic capture beads which are now binding the target analyte and control analyte out of suspension as shown in FIG 15. The magnet 130 on the bottom side of the cartridge is brought into proximity of the surface of the cartridge in a preferred location to pull the beads into a tight bolus. The bead bolus may be imaged by a processing quality control camera and analyzed to ensure the beads have been properly pulled from suspension.

### Introduction of Wash Buffers and Washing

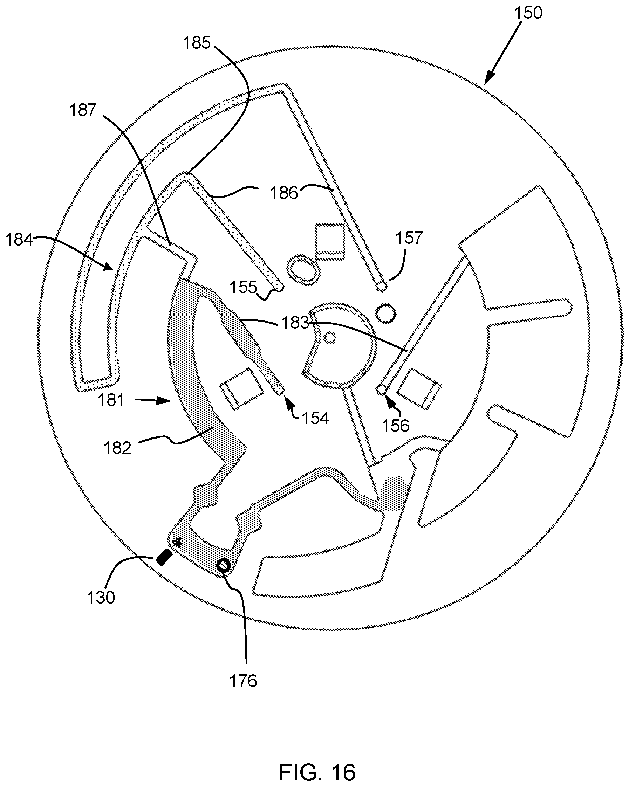

At this point the wash valve connects the wash pump to the wash entry port 154 on the cartridge and the draw pump is connected to the wash draw port 156. The other ports remain closed. The wash pump and draw pump are controlled to empty and draw respectively at defined volumetric flow rates. This causes wash buffer to fill the wash chamber and the channel leading to the mix chamber as shown in FIG. 16. The sequence continues until the supernatant is pushed out of the mix chamber and back into the separation chamber. This serves to wash much of the unbound labels and contaminates away from the bead bolus which is held in place by the magnet. Once the wash buffer fill sequence has completed, an image of the wash chamber may be collected from a processing quality control camera and processed to ensure complete proper filling of the wash chamber, channel and mix chamber with wash buffer.

### Magnetic Transfer for Washing

At this point the magnet stages and manifold motors execute a sequence of motions to drag the paramagnetic bead bolus out of the mix-incubation chamber and radially along the channel between the wash and mix chamber into the wash chamber. At various points in this process imagery may be captured and processed to ensure that the bead bolus is of appropriate size and that the process has executed in a manner to ensure all of the bead bolus has been pulled into the wash chamber.

### Wash Sequence

When the bead bolus has been transferred to the wash chamber the manifold motor, magnet Z-stage, and radial stages are controlled to execute a sequence of motions to disperse the beads and re-condense the bead bolus using the magnets alternately on different sides of the wash chamber along the length of the wash chamber. This process is done to remove any remaining unbound label or other contaminates that may been trapped in the bead bolus. When the wash sequence is complete, the paramagnetic capture beads are again pulled into a tight bolus, Clean wash buffer is then pumped into the wash chamber while the contaminated buffer is pushed out through the mixing chamber and into the wash waste chamber 166 located on this cartridge. The wash waste chamber 166 is sized such that the contaminated wash, original sample and supernatant never completely fill the wash waste chamber and exit through the wash draw port located at the far end of the wash waste chamber. This ensures the manifold never comes into contact with any sample or with any buffers that may have mixed with the sample.

### Air Dam Removal and Elution Buffer Fill

The wash chamber 181 is connected to an elution chamber 184 at the end opposite the mixing chamber via a connecting passage 187. The elution chamber 184 is also connected to the elution port 155 and elution draw port 157. When the wash chamber is filled, as previously discussed, the sequence is carried out in a manner to deter wash buffer from entering the channel and the elution chamber 184. This is important because contaminated wash buffer contains unbound label and other elements that might be sources of noise during subsequent measurement. A sufficient air gap between the wash chamber 181 and elution chamber 184 may be confirmed via analysis of images taken by the processing quality control camera during each wash fill sequence. After the last magnetic wash sequence and after the wash chamber is again filled with clean wash buffer, a sequence is conducted to fill the air gap (if desired) in connecting passage 187 between the wash chamber and elution chamber 184 with wash buffer. The wash draw port valve is closed, the elution draw port is opened, and the draw pump is connected to the elution draw port 114. A pump sequence is executed with the wash and draw pumps at predefined volumetric flow rates to fill the channel between the wash and elution chambers. The process may be confirmed via image collection and analysis.

When complete, another sequence is carried out wherein the elution pump fills the elution chamber with elution buffer. Image processing may be performed to ensure the channel and elution chamber fill correctly. At this point a pre-read of the pure elution buffer, prior to sample introduction into the elution chamber, may be performed in the same manner that the processed sample will be read to serve as a negative control for the assay. The elution buffer is designed to cleave the bonds between the target analyte and its associated label as discussed below. It is important that this process be avoided when the sample bead bolus is outside the elution chamber.

### Magnetic Transfer to Detection Chamber

Once the elution chamber is filled with elution buffer the magnet stages and manifold motors are controlled in a predefined sequence to pull the bead bolus from the wash chamber 181 through the connecting passage 187 into the elution chamber 184.

### Preparation for Measurement

Once the bead bolus is in the elution chamber, a process similar to the wash process is conducted in the elution chamber. While the wash steps left contaminates suspended and to be washed away, the elution process is designed to leave the labels which were once bound to the target analytes (and control analyte) dissociated and in suspension. The combined movement of the upper and lower magnets combined with precise rotary movement of the cartridge causes the bead bolus to repeatedly disperse and re-condense across the length of the channel. The elution buffer cleaves the non-covalent bonds between the target analyte and the paramagnetic capture beads as well as the bonds between the target analyte and its label, which results in a homogeneous solution of eluted labels in the elution chamber. The same cleaving occurs for the control analyte. The entire purification process described briefly herein is designed to create a suspension containing only the isolated target analyte and the labels that were once bound to that target analyte in a one to one relationship. The paramagnetic capture beads which were used to capture the target analyte are a source of noise for the read process. After the elution sequence in which the target analyte bonds are cleaved, the dissociated paramagnetic capture beads may be pulled to a preferred location in the elution chamber via the magnets and away from where the processed sample will be read.

### Measurement

In the measurement step, a confocal laser-based optical system is focused within the elution chamber, for example at a point in the elution chamber away from the walls, upper and lower surfaces of the chamber. Measurement and detection of analyte occurs in the elution chamber 184, therefore chamber 184 serves as both the elution chamber and the detection chamber and both terms are used throughout the disclosure to refer to chamber 184. The cartridge itself may be made of ultra-low autofluorescence material and the elution buffer, pump materials, valves, fluidic lines etc. may be selected such that they do not shed or leach materials which might autofluoresce if carried into the elution chamber. A small interrogation space is scanned through the liquid in the elution chamber by spinning the cartridge at a predefined rpm back and forth via the manifold motor. The interrogation space is defined by the lateral extent of the laser spot and the lateral extent of the cone angle of light forming the laser spot. The interrogation space is further defined along the optic axis by the size of the confocal stop positioned conjugate to the field in the optical system. As those skilled in the art will appreciate a confocal architecture is used to remove light from positions away from the focal plane. The further away from the focal plane and the smaller the confocal stop, the more the light coming from distant positions is attenuated. In imaging applications, this reduction in out-of-focus light reduces noise and provides crisp image slices. Light coming from positions away from the focal plane (or image slice) does not represent the structure in the image slice and is therefore noise. The same process of noise reduction may be employed in the present disclsoure; however, in this case the confocal system is not used for imaging. As the laser spot scans through the fluid it may encounter a fluorescent label from the target analyte. When it does, the laser excites fluorescence from the label and individual photons are emitted from the label and directed by the optical system on to a detector where they are counted. On the way to the focal plane, and beyond the focal plane, the laser light may encounter elements that autofluoresce, including the glasses and bonding materials that comprise the optical system, the window on the cartridge, the back side of the elution chamber, the elution buffer and any other material that might have made it into the elution chamber. Any fluorescence from those components is noise since it is not from a target analyte label. The confocal architecture attenuates those signals by preferentially allowing signal from target analyte labels in or near the focal plane. As a result, when the laser passes over a target label, the stream of photons received and counted by the detector increases over the background photon level. Processing algorithms detect and classify the elevated photon count as a molecule of interest. In this manner individual molecules from the target analyte can be counted to determine a concentration of the target analyte in the original sample.

The present disclosure enables substantial advantages over the prior art to precisely detect and quantify the number of target analytes in a sample wherein the concentration of target analytes in the sample is low. Further, the methods and apparatus of the present disclosure have characteristics suitable for deployment in a point-of-care setting. Also described herein are methods of sample processing and analysis to isolate target analytes and determine their concentration. These methods implement steps that are generally consistent with the sample processing and measurement above.

### Examples

Examples and systems are described herein. It should be understood that the words "example" and "exemplary" are used herein to mean "serving as an example, instance, or illustration." Any embodiment or feature described herein as being an "example" or "exemplary" is not necessarily to be construed as preferred or advantageous over other embodiments or features. In the following detailed description, reference is made to the accompanying figures, which form a part thereof. In the figures, similar symbols typically identify similar components, unless context dictates otherwise. Other embodiments may be utilized, and other changes may be made, without departing from the scope of the subject matter presented herein.

The example embodiments described herein are not meant to be limiting. It will be readily understood that the aspects of the present disclosure, as generally described herein, and illustrated in the figures, can be arranged, substituted, combined, separated, and designed in a wide variety of different configurations, all of which are explicitly contemplated herein.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

Reference herein to "one embodiment," "an embodiment," "one example," or "an example" means that one or more feature, structure, or characteristic described in connection with the example is included in at least one implementation. The phrases "one embodiment" or "one example" in various places in the specification may or may not be referring to the same example.

As used herein, a system, apparatus, device, structure, article, element, component, or hardware "configured to" perform a specified function is indeed capable of performing the specified function without any alteration, rather than merely having potential to perform the specified function after further modification. In other words, the system, apparatus, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware which enable the system, apparatus, structure, article, element, component, or hardware to perform the specified function without further modification. For purposes of this disclosure, a system, apparatus, structure, article, element, component, or hardware described as being "configured to" perform a particular function may additionally or alternatively be described as being "adapted to" and/or as being "operative to" perform that function.

In the following description, numerous specific details are set forth to provide a thorough understanding of the disclosed concepts, which may be practiced without some or all of these particulars. In other instances, details of known devices and/or processes have been omitted to avoid unnecessarily obscuring the disclosure. While some concepts will be described in conjunction with specific examples, it will be understood that these examples are not intended to be limiting.

### Example Analyzer System

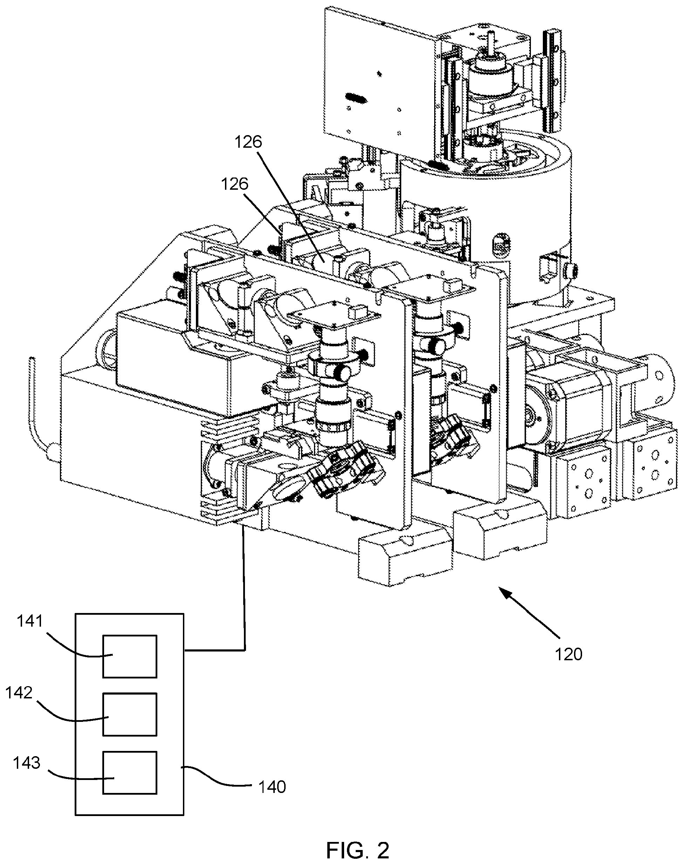

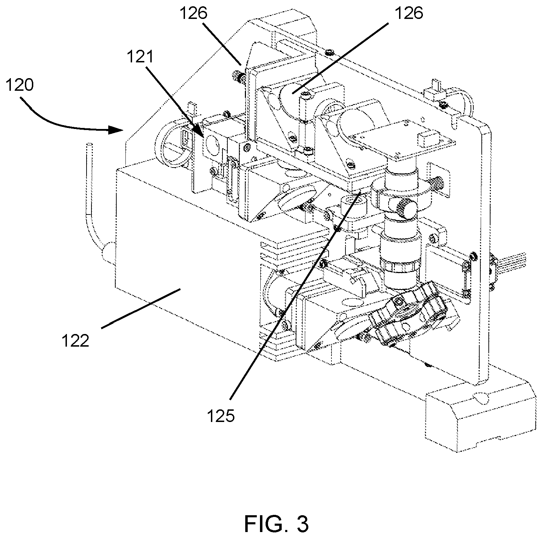

Described herein is an analyzer system shown in FIG. 1 that includes an analyzer 100 and a cartridge 150. The cartridge 150 is configured to receive a sample and includes a plurality of chambers for isolating a target analyte of the sample and collecting a quantity of a first label that is proportional to a quantity of the target analyte in the sample. The analyzer 100 includes an optical system 120, which is more clearly seen in the rear view of the analyzer 100 in FIG. 2. Further, components of the optical system 120 are shown separately from other parts of the analyzer in FIG. 3, for clarity. As shown, optical system 120 includes an electromagnetic radiation source 121 configured to provide electromagnetic radiation to form an interrogation space within a detection chamber of the cartridge 150. The optical system 120 also includes a detector 122 configured to detect electromagnetic radiation emitted in the interrogation space by the first label if the first label is present in the interrogation space. Other components of the optical system 120 are described in more detail below.

Analyzer 100 also includes a controller 140, which is schematically represented in FIG. 1. The controller 140 includes a non-transitory computer-readable medium with program instructions stored thereon for carrying out the steps conducted by the analyzer 100 and identifies the presence of the target analyte in the sample based on the electromagnetic radiation detected by the detector 122. Controller 140 includes a processor 141, a memory 142, and a network interface 143.

Processor 141 of controller 140 includes a computer processing elements, e.g., a central processing unit (CPU), an integrated circuit that performs processor operations, a digital signal processor (DSP), or a network processor. In some disclosures, the processor includes register memory that temporarily stores instructions being executed and corresponding data, as well as cache memory that temporarily stores performed instructions. Memory 142 is a computer-usable memory, e.g., random access memory (RAM), read-only memory (ROM), or non-volatile memory such as flash memory, solid state drives, or hard-disk drives. In some disclosures, memory 142 stores program instructions that are executable by controller 140 for carrying out the methods and operations of the disclosure. Network interface 143 provides digital communication between controller 140 and other computing systems or devices. In some disclosures, the network interface operates via a physical wired connection, such as an ethernet connection. In other disclosures, the network interface communicates via a wireless connection, e.g., IEEE 802.11 (Wifi) or BLUETOOTH. Other communication conventions are also possible.

In some disclosures, the analyzer 100 includes at least one motor configured to rotate the cartridge in order to manipulate any sample placed in the cartridge and to align the cartridge with parts of the analyzer. In some disclosures, the motor is a centrifuge drive motor and in other disclosures the motor is a positioning motor. Further, in some disclosures, the analyzer includes both a centrifuge and a positioning motor. For example, analyzer 100, shown in FIG. 1, includes both a centrifuge 101 and a positioning motor 110.

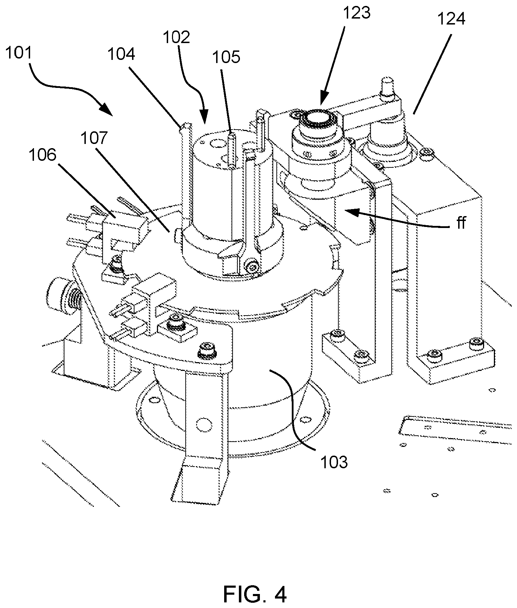

In analyzer 100 the centrifuge 101 is coupled to the cartridge 150 in order to spin the cartridge at a speed of at least 100 rpm. Details of the centrifuge 101 are shown more clearly in FIG. 4. As illustrated, a centrifuge drive motor 103 is configured to couple to the cartridge using a dock 102. The dock 102 may include a retaining mechanism, such as a plurality of registration pins 105 and cantilevered clips 104 that mate with mounting apertures 153 of the cartridge 150 (shown in FIG. 9). In some disclosures, the cantilevered clips 104 are at the end of leaf springs that clamp over the cartridge when the cartridge 150 is received in the dock 102. The cantilevered clips 104 may be configured to engage the cartridge 150 in an outward direction. As the centrifuge 101 spins, the cantilevered clips 104 are urged outward by centrifugal force increasing the holding force on the cartridge 150. Accordingly, the cartridge 150 may be securely held in the dock 102 when inserted into the analyzer 100.

The dock 102 is connected to the centrifuge drive motor 103 in order to rotate the dock 102 and the cartridge 150 that is attached thereto. Further, the centrifuge drive motor 103 may include electronic drive phase sensors 106 and a flag wheel 107 for high-speed precision control of the centrifuge 101 during operation. In some disclosures, the dock 102 is driven directly by the centrifuge drive motor 103, while in other disclosures, a power transfer system, such as a gearbox or a belt drive, may be used to couple the dock 102 to the centrifuge drive motor 103. As explained below, the dock 102 may also be driven by a manifold 108 (see FIG. 1). Specific disclosures of operations of the centrifuge 101 are described in more detail below.

In some disclosures, the analyzer includes a manifold 108 with a plurality of ports that are each configured to couple to a respective port of the cartridge. A depiction of the manifold 108 coupled to the cartridge 150 is shown in FIG. 5. Further, a bottom view of the manifold 108 is shown in FIG. 6 to illustrate the ports 111 to 114. In some disclosures, the manifold includes supply ports 111, 112 for supplying fluid to the cartridge 150 and draw ports 113, 114 for drawing fluid from the cartridge 150. In some disclosures, the supply ports 111, 112 are used to provide one or more liquids to the cartridge 150, while the draw ports 113, 114 draw the liquid into the cartridge 150 by extracting gas from the cartridge 150. In other disclosures, the draw ports extract liquid, or both liquid and gas, from the cartridge 150. In order to transfer fluids to and from the cartridge, the manifold 108 includes fluidic lines 115 that are connected to the ports 111 to 114.

Each of the ports of the manifold 108 may include a seal that covers the respective corresponding port of the cartridge 150 in order to isolate the fluid transfer between the manifold 108 and the cartridge 150. For example, each of the ports 111 to 114 may include an O-ring or other feature to create a seal between the manifold and cartridge ports that surrounds the respective port of the cartridge 150. In some disclosures, the ports of the cartridge 150 are already open when the cartridge 150 is placed in the analyzer. In other embodiments the manifold is configured to pierce the cartridge 150 so as to open each of the ports of the cartridge 150.

In some disclosures, the manifold 108 is disposed on a movable arm 109 (shown in FIG. 1), which allows the manifold 108 to be decoupled from the cartridge 150 when the cartridge 150 is rotated at high speeds by the centrifuge 101. Coupling of the manifold 108 to the cartridge 150 may be enabled by an alignment structure. For example, the manifold 108 may include pins that are received in mounting apertures 153 of the cartridge (see FIG. 9). In other disclosures, the pins 104 of the dock 102 may pass through the mounting apertures 153 of the cartridge 150 into receiving holes in the manifold 108. Such a structure provides a secure connection between the manifold 108, the cartridge 150 and the dock 102. Other mounting structures are also possible, as will be appreciated by those of ordinary skill in the art.

In some disclosures, the analyzer 100 includes a positioning motor 110 that is coupled to the cartridge 150. In some disclosures the positioning motor 110 may be coupled to the manifold 108 which couples to the cartridge 150. The positioning motor 110 may be configured to pivot the cartridge 150 so as to align the detection chamber 184 (see FIG. 9) of the cartridge 150 with the electromagnetic radiation from the first electromagnetic radiation source 121. Moreover, the positioning motor 110, in concert with one or more magnets or by the fluid dynamics of the sample, may further be used to circulate the target analytes through the chambers of the cartridge 150, as described in more detail below. The positioning motor 110 may be a stepper motor or another actuator with specific positioning control. For example, in some disclosures, the location of the positioning motor 110 may be specified to within 2° of rotation, or within 1° of rotation, or smaller than 1° increments. Specific examples of disclosures of using the positioning motor 110 are described in more detail below.

In some disclosures, the positioning motor 110 is directly coupled to the manifold 108, while in other disclosures, a power transfer system, such as a gearbox or a belt drive, may be disposed between the positioning motor 110 and the manifold 108. In the analyzer 100 shown in FIG. 1, the positioning motor 110 is coupled to the cartridge 150 via the manifold 108. In particular, manifold 108 is disposed on the shaft of the positioning motor 110. Accordingly, the manifold 108 and cartridge 150 may move synchronously while maintaining a closed fluid connection therebetween.

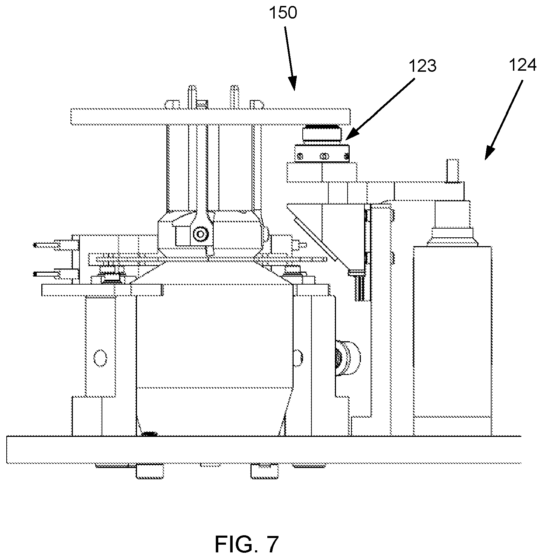

In some disclosures, the analyzer 100 includes an optical system 120 (FIGS. 2-3) that directs the electromagnetic radiation from the first electromagnetic radiation source 121 to the detection chamber 184 of the cartridge 150, and that directs the electromagnetic radiation emitted by the label to the first detector 122. The optical system 120 may include one or more mirrors and lenses to manipulate and direct the electromagnetic radiation to and from the interrogation space. In addition, the optical system may include an objective 123, as shown in FIG. 7, for focusing the electromagnetic radiation from the first electromagnetic radiation source to the interrogation space in the cartridge 150. In some disclosures, the objective 123 is coupled to a movable stage 124, which allows movement of the objective with respect to the cartridge 150.

In some disclosures, the optical system 120 is a confocal system. For example, the electromagnetic radiation source 121 is imaged as a spot in the focal plane of the objective lens 123 within the detection chamber 184. Light emitted from a label in the detection chamber 184 excited by the electromagnetic radiation source 121 is collected by objective lens 123 and directed by the optical system 120 onto a confocal stop 125 in the optical system 120 as shown in FIG. 3. The confocal stop 125 is then imaged onto the detector 122. The confocal arrangement preferentially passes light from the label in the focal plane of the objective 123 while excluding light from beyond the focal plane. In this manner the arrangement increases the signal to noise ratio by passing signal from the label while excluding light from elements in the liquid suspension, cartridge and optical system that are not originating from the label. As is known to those skilled in the art, this arrangement may also use a dichroic filters 126 to reflect laser light and pass light emitted by the label to only allow light from the label to reach the detector while prohibiting laser light from reaching the detector. Further, if more than one radiation source is used for detection of additional labels than one or more additional dichroic filters 126 may be used to reflect laser and label electromagnetic radiation from the first electromagnetic radiation source and label while passing electromagnetic radiation from a second electromagnetic radiation source and second label as shown in FIG. 2 and FIG. 3.

In some disclosures all of the components of the analyzer 100 are disposed in a common housing. The common housing may be small in size, so as to fit on a countertop. For example, in some disclosures, the dimensions of the common housing are no greater than 1 meter in any direction. Further, in some disclosures, the common housing fits within a 30 inch x 30 inch x 30 inch cube.

In some disclosures the controller 140 includes a network interface 143 for receiving control information from a user and for outputting analysis data to the user. For example, in some disclosures, the analyzer communicates with a user via software on an external device, such as a smartphone, table, notebook computer, or desktop computer. The analyzer receives information from and outputs information to the user of the external device by communicating with the external device via the network interface. Such communication may be through a wireless or wired connection, such as a USB or other bus. In some disclosures, the analyzer 100 may include an input and/or output devices for communicating directly with a user, such as a keyboard for receiving inputs and a display for outputting information. Moreover, in some disclosuresthe display may include a touchscreen for both outputting information and receiving information from a user. In some disclosuresthe analyzer includes a network interface, an input, and a display.

In some disclosuresthe method of the disclosure includes directing portions of the sample through the chambers of the cartridge 150 without the cartridge 150 including any valves. Further, in some disclosures, the cartridge 150 is free of any valves.

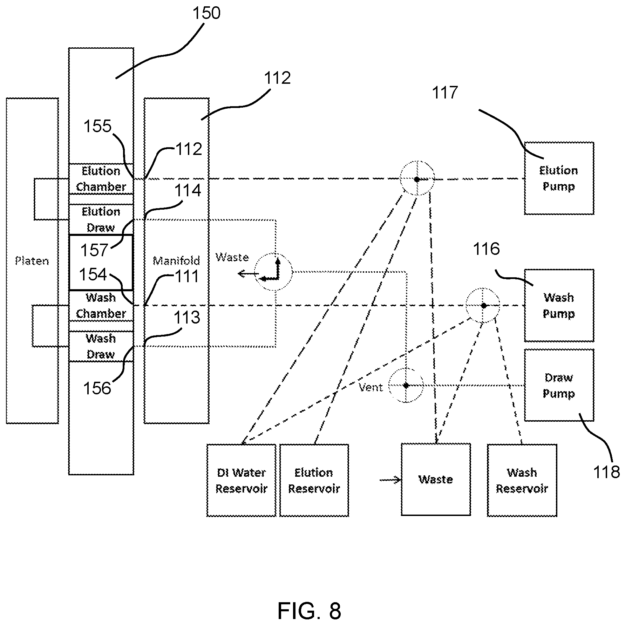

In some disclosures, liquids within the cartridge 150 are, at least in part, moved through the cartridge using pumps 116- to 118 and valves coupled to the supply and draw ports of the cartridge, as described in more detail below. A schematic view of fluid transfer components of the analyzer 100 is shown in FIG. 8. Manipulation of portions of the sample within the cartridge 150 may also be facilitated using external motive forces, such as magnets, or by movement of the cartridge 150 and utilizing inertia and fluid dynamics to move portions of the sample around the cartridge.

As shown in FIG. 8, in some disclosures, the cartridge 150 includes a plurality of ports 154 to 157 for introducing and extracting fluid from the cartridge 150. For example, in some disclosures, the cartridge 150 includes inlet ports 154, 155 and outlet ports 156, 157. The inlet ports 154, 155 may be configured to align with the supply ports 111, 112 of the manifold 108. Likewise, the outlet ports 156, 157 of the cartridge 150 may be configured to align with the draw ports 113, 114 of the manifold 108. Use of the inlet and outlet ports of the cartridge 150 is described in greater detail below.

As described herein, the disclosure provides a plurality of chambers for isolating a target analyte of the sample and collecting a quantity of a first label that is proportional to the concentration of the target analyte in the sample.

In some disclosures, the cartridge 150 is planar and the chambers of the cartridge lie in a single plane. For example, in some disclosures, the cartridge 150 is a flat cartridge and the chambers of the cartridge are positioned circumferentially around the cartridge. The term circumferentially, as used herein, refers to the angular or circumferential direction, as opposed to a radial or axial direction. Unless otherwise stated, the term circumferentially is not intended to mean extending about the entire circumference of the cartridge, but rather to denote the circumferential direction in the plane of rotation. In some disclosures, at least a group of the chambers may be sequentially connected circumferentially around a portion of the cartridge.

In some disclosures, the cartridge may include a base, a body disposed over the base, and a cover disposed over the body, where the body includes an open path extending therethrough that defines the plurality of chambers of the cartridge 150. In some disclosures, the body may be a single integral piece. Thus, for example, in some disclosures, the side walls of all of the chambers and interconnecting channels of the cartridge may be formed by a single integral piece that forms the body. Moreover, in some disclosures the body and the base together form a single integral piece and the cover is attached thereto. Likewise, in other disclosures, the body and the cover form a single integral piece, and the base is attached thereto. For example in this disclosure, the body and base may be a single molded piece of cyclic olefin polymer that is 5mm thick and the cover may be a laminate of cyclic olefin polymer that is 188 microns thick. The laminate may be bonded to the body using laser welding or ultrasonic welding to provide a bond that is as strong as the materials being bonded together. In some disclosures, the base, the body, and the cover may be layers of a laminate structure. For example, in some disclosures, the base and the cover are both laminated over opposing sides of the body. In some disclosures, the cover and base of the cartridge 150 extend over and close the chambers and microfluidic channels of the cartridge, although they may include ports, as described above, to supply or extract fluids from the cartridge.

In some disclosures, the cartridge is configured to receive a sample in a range of 50 microliters to 1 milliliter. For example, in some disclosures, the cartridge is configured to receive a sample in a range or 100 to 300 microliters. In particular, the cartridge may include a metered chamber for receiving the sample.

In some disclosures, the cartridge includes reagents stored within at least one of the plurality of chambers. For example, in some disclosures, the cartridge includes reagents that are stable and dried before the cartridge is inserted into the analyzer. For example, the reagents may be lyophilized or dried onto the surface of one or more chambers of the cartridge. Or they may be in the form of lyophilized pellets placed into one or more of the chambers or the cartridge.

While the cartridge is shown and described herein in the form of a disc that spins within the analyzer, it is contemplated but not claimed that in some cases the cartridge is not a disc or may not be used at all. For example, parts of the disclosure may be carried out in discrete separate elements that form the different chambers.

### Processing Quality Control Camera

As described herein, the analyzer 100 includes a processing quality control camera for monitoring the movement of substances through the cartridge 150. For example, the processing quality control camera may be mounted over the cartridge 150 so as to view the substances inside the cartridge 150. In some disclosures, the processing quality control camera is configured to output a representation of only light detected in the visible wavelength spectrum, i.e. the camera is not enabled to detect infrared or ultraviolet light. In some disclosures, the controller 140 is configured to analyze images from the processing quality control camera so as to confirm that the sample processing occurs as expected or to detect any unexpected circumstances. For example, the controller 140 may be configured to detect the presence of an undesired air bubble in the cartridge. Other example disclosures of using the processing quality control camera are described below.

As described herein, the analyzer includes a strobe that is positioned to illuminate the field of view of the processing quality control camera. For example, the strobe may be configured to activate at a frequency that corresponds to the rotational speed of the cartridge 150, in order to monitor a specific region of the cartridge 150 as it is rotated. In particular, in some embodiments the strobe may be used when the centrifuge 101 is rotating the cartridge 150.

### Optics Quality Control Camera

As described herein, the analyzer 100 includes an optics quality control camera for monitoring the performance of the optical system 120. For example, the optics quality control camera may use a mirror on a slide to intercept the optical path before after the confocal stop to image the laser at the confocal stop in order to visualize that the electromagnetic radiation has the appropriate intensity, is focused in the correct location, and or has the correct intensity profile. In order to image the laser at the confocal stop the objective may be positioned so that the electromagnetic radiation source is imaged onto the surface of a window on the cartridge 150. When this is done a portion of the radiation will reflect off the window back towards the objective due to the difference in the index of refraction of the window and the media on the other side of the window. This radiation will be imaged by the optical system onto the confocal stop. The window on the cartridge may be sized of the correct thickness to simulate the thickness of the window of the detection chamber and height of the fluid layer between the window and focused spot of electromagnetic radiation. The image of the electromagnetic radiation at the confocal step can be analyzed by the controller 140. The controller 140 may be used to analyze the images from the optics quality control camera to verify that the electromagnetic spot is of the correct size, shape, intensity and position relative to the confocal stop to ensure there are no anomalies in the optical system. The measured size, shape, intensity and position can be compared to known and accepted values for these parameters. If the measured values are outside accepted values or approaching the limits of accepted values, the controller can notify the user of the analyzer or prevent usage of the analyzer.

### Example Method

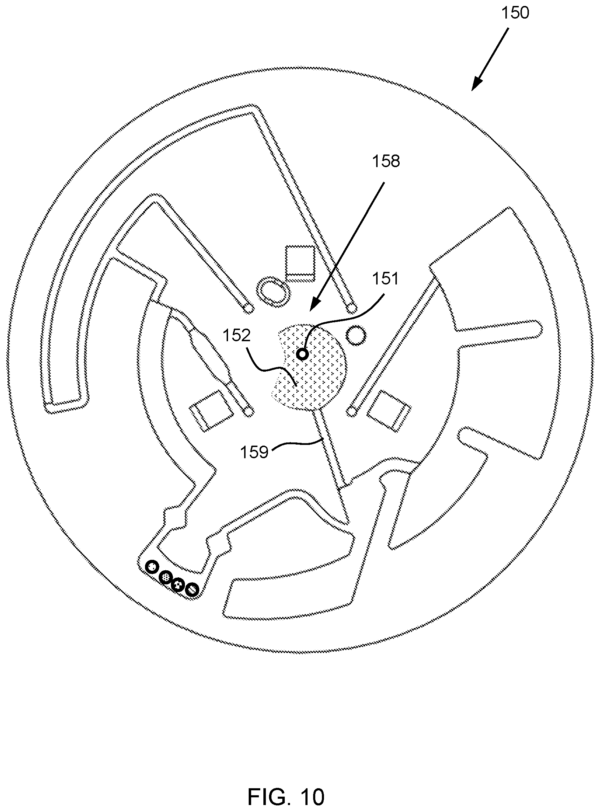

FIGS. 10 to 18 illustrate an example cartridge and method that utilizes various disclosures where the sample is blood. In other disclosures the chambers of the cartridge and methods used may be suited for other sample types. For example, analyzers, methods and cartridges of the disclosure may be suited for use with other biological fluids, such as urine, diluted stool or oral fluid. Other types of samples are also possible. Further, the samples may be neat or diluted.

### Loading and Sample Separation

As shown in FIG. 10, cartridge 150 is initially loaded with a sample 152 in an inlet chamber 158. The inlet chamber 158 includes an input port 151 that receives the sample 152 prior to analysis. In some disclosures, the sample 152 is received in the cartridge 150 prior to insertion in the analyzer 100, for example by a medical professional or robot that uses a syringe. In other disclosures, the inlet chamber 158 is loaded with the sample 152 after the cartridge 150 is received in the analyzer 100. As mentioned above, in some disclosures the input port 151 can be sealed prior to insertion of the sample 152, and the seal can either be pierced or removed to enable insertion of the sample 152. In other disclosures, the input port 151 can be a simple opening that is available to receive the sample 152 without being "opened." In some disclosures the input port 151 can be sealed after the sample has been input. In other embodiments the manifold 108 contains a seal to cover the port when the manifold is in contact with the cartridge. In some disclosures , the inlet chamber 158 is a metered chamber configured to receive a specific amount of sample, while in other disclosures , the inlet chamber 158 is oversized and can accommodate more sample than is used in the analysis. The inlet chamber 158 in the illustrated example of FIGS. 9 to 18 is configured to receive about 200µl of liquid.

Once the sample 152 is loaded into the inlet chamber 158, as shown in FIG. 10, and the cartridge 150 is inserted into the analyzer 100, the cartridge 150 is coupled to the centrifuge 101 so that the centrifuge 101 may spin the cartridge 150. As explained in more detail below, the geometry of the chambers and channels within the cartridge 150 are designed to influence the transfer of fluid through the cartridge 150. In order to facilitate an understanding of these geometries, the following description makes reference to cylindrical/polar directions. In particular, use of the terms "inner," "inward", "outer", "outward" and similar descriptors refer to a radially inner and radially outer direction with respect to the center of rotation of the cartridge, which typically lies near the geometric center of the cartridge. The description also references a first circumferential direction and a second circumferential direction, which are related to the direction that the cartridge is configured to be spun by the centrifuge, where the cartridge is configured to be spun in the first circumferential direction. For example, an area at a first circumferential end of a chamber will pass a stationary reference position before an area at the second circumferential end of the same chamber. In the disclosures shown in FIGS. 9 to 18, the first circumferential direction is clockwise, however other disclosures of the cartridge may be configured to spin in the opposite directions, such that in these disclosures the first circumferential direction is counter-clockwise.

With the cartridge 150 loaded in the analyzer 100, the centrifuge 101 is activated to rotate the cartridge 150 in order to move the sample 152 from the inlet chamber 158 through an inlet channel 159 into a separation area 160, as shown in FIG. 11. The rotation of cartridge 150 causes the sample 152 to move radially outward as a result of "centrifugal force," i.e., the inertial phenomenon that causes objects to move outward when rotated. If the sample volume is greater than the amount needed for analysis, any excess may flow out of the separation area 160 through an overflow channel 165. In some disclosures, the inlet chamber 158 may be offset from the center of the cartridge 150 to facilitate the transfer of the sample to the separation area 160. In other disclosures, the inlet chamber 158 is located at the center of the cartridge 150 so that rotation of the disc-shaped cartridge 150, once loaded with the sample, will keep the sample and any other liquids received in the cartridge 150 away from the input port 151. Further, in some disclosures, input port 151 may be centered on the cartridge 150. In some disclosures to move the sample from the inlet chamber 158 to the separation area 160 the cartridge may for example, be spun up from 0 rpm to 1000 rpm at a rate of 2000 rpm/s and held at that speed for a couple seconds, for example 2-10 seconds. Thus the sample transfer may occur very quickly. The rotation rates and accelerations provided are exemplary and the actual rates chosen will depend on the sample being processed and may vary in rotation from 100 to 10,000 rpm with accelerations varying between 100 rpm/s and 8000 rpm/s.

In some disclosures, the separation area may include an inner separation chamber 161 and an outer separation chamber 162 configured to hold the different constituents of the sample after they are separated. In some disclosures the center of the inner separation chamber may be located at 19mm from the center of rotation and the center of the outer separation chamber may be located at 28mm from the center of rotation. As the centrifuge spins the cartridge 150, denser constituents of the sample are pushed radially outward into the outer separation chamber 162, while the less dense constituents move radially inward into the inner separation chamber 161. In some disclosures, the inner and outer separation chambers 161, 162 of separation area 160 are separated by a constricted neck 163 located for example at 22mm from the center of rotation. The constricted neck 163 has a smaller cross-sectional area than either of the chambers. For example, in some disclosures the constricted neck 163 may have a cross sectional area of 3mm² while the inner separation area 162 has an average cross-sectional area of 12mm² and the outer separation area 162 has an average cross sectional area of 30mm². In this example disclosure the neck 163 is sized to readily allow more dense constituents to move downward while less dense constituent move upward through the neck 163 quickly. However, as is discussed below the constricted neck 163 limits the movement of more dense constituents into the inner separation area 161 when the cartridge is rapidly decelerated.

In order to generate an accurate concentration value of the sample for further processing, the precise volume of the sample should be known. If the sample is unable to fill the separation area 160 and is unintentionally wasted, or if the separation area is sized to accept more than the sample volume, accurate concentration values might be difficult to obtain. Therefore, in some disclosures the cartridge 150 may include various features for metering a precise amount of fluid into the separation area 160.

For example, some disclosures of the cartridge 150 may include one or more features to avoid the trapping of air in the cartridge, particularly during the transfer of the sample from the inlet chamber 158 to subsequent chambers. If air is trapped in the separation area 160 as the sample is loaded therein, some of the sample may prematurely flow through the overflow channel 165 and precise metering of the sample into the separation area 160 may be unsuccessful. Accordingly, avoiding the formation of trapped air in the cartridge during loading is beneficial.

In some disclosures, the inlet channel 159 is coupled to a first circumferential end of the inner separation chamber 161. As the sample moves outward from the inlet chamber 158 through the inlet channel 159 and into the separation area 160, the rotation and/or acceleration of the cartridge 150 in the first circumferential direction by the centrifuge 101 can cause the sample to flow in the second circumferential direction. Accordingly, if the inlet channel 159 is coupled to the middle of the inner separation chamber 161, additional precautions may be necessary to avoid the formation of trapped air in a corner at the first circumferential end toward the inner side of the inner separation chamber 161. However, if the inlet channel 159 is coupled to the first circumferential end of the inner separation chamber 161, as shown in the cartridge 150 of FIGS 9 to 18, the inclusion of an inner corner that is further in the first circumferential direction than the inlet channel 159 opening is avoided. Likewise, air that might be trapped in such a corner is also avoided.

Further, in some disclosures, the inlet channel 159 may be constricted in size and depth compared to the separation area 160. Such a constriction can slow the flow of sample into the separation area 160, allowing air to be purged from the separation area 160 while it is filling. Furthermore, the constricted size and depth may also help avoid the formation of a sheet of liquid across a cross section of the separation area 160, which could also form trapped air. For example, in one disclosure the depth of the inlet channel 159 may be 0.5mm while the depth of the inner separation chamber 161 is 2mm. Accordingly, the stream of sample flowing into the inner separation chamber 161 from the inlet channel 159 will not span the entire depth of the inner separation chamber 161, allowing air to flow around the stream and out of the separation area 160.

Further, in some disclosures, the cross-sectional area of the inlet channel 159 may be narrower than the cross-sectional area of the constricted neck 163 between the inner separation chamber 161 and the outer separation chamber 162. For example the inlet channel 159 may have a cross sectional area of 0.5 mm² while the constricted neck 163 has a cross sectional area of 3mm². Accordingly, the volumetric flow rate of the sample into the separation area 160 is unlikely to overwhelm the constricted neck 163 and trap air in the outer separation chamber.

To prevent the trapping of air in the outer separation chamber 162, in some disclosures, the inner edge 164 of the outer separation chamber 162 extends at an angle projecting inward as the inner edge 164 approaches the constricted neck 163 that separates the inner separation chamber 161 from the outer separation chamber 162. Accordingly, as the outer separation chamber 162 fills with the sample due to the rotation of the cartridge, air in the outer separation chamber 162 will "float" inward to the inner edge 164 and then follow the inner edge 164 to the constricted neck 163. The air will then pass through the constricted neck 163, through the inner separation chamber 161 and out of the separation area 160.

In some disclosures, the controller 140 is configured to capture an image of the separation area 160 or a portion thereof using the processing quality control camera after the separation area 160 is filled. The controller may further be configured to analyze the image to confirm that the volume of any air bubbles within the separation area 160 is void of any air bubbles or that the volume of air in the separation area is below a predetermined threshold. For example, the controller may be configured to calculate the shape of any air bubbles within the separation area 160 and calculate the overall volume of air within the separation area 160. If the calculated volume of air is above a predetermined threshold, the controller may be configured to discontinue the analysis. Likewise, the controller may be configured to continue the analysis if the calculated volume of air is below a predetermined threshold or is zero.

In some disclosures, the separation area 160 and surrounding channels may include one or more features for precise metering of the sample and controlled separation of components of the sample. For example, in some disclosures, the overflow channel 165 may be positioned to enable precise metering of the amount of sample 152 into separation area 160. If the amount of sample 152 received in the cartridge 150 is more than needed for the analysis, the excess will discharge through the overflow channel 165. In some disclosures, the overflow channel 165 leads to a waste chamber 166 where the excess liquid may be stored.

Due to the rotation of the cartridge 150 and the centrifugal force on the sample, the separation area 160 fills from the outer end toward the inner end. Accordingly, positioning the opening of the overflow channel 165 at a particular radial position in the inner separation chamber 161 dictates the quantity of sample that can be loaded into the separation area 160. For example, as the centrifuge 101 spins the cartridge 150 the sample will move toward the outer end of the outer separation chamber 162 and produce a fill line that moves inward as the separation area 160 fills. Once the fill line reaches radial position of the overflow channel 165, for example at a radial distance of 17mm, any additional volume of sample that enters the separation area 160 will exit the separation area 160 through the overflow channel 165. Therefore, the quantity of the sample that will be analyzed can be precisely metered based on the radial position of the overflow channel 165.

### Separation of Sample Constituents

As shown in FIG. 12, after the sample has been loaded into the separation area 160, the centrifuge 101 may continue to spin the cartridge 150 in order to separate the sample 152 into different constituents. For example, the centrifuge 101 may spin the cartridge 150 so as to send denser constituents of the sample outward leaving less dense constituents radially inward. In some disclosures, the speed of the centrifuge 101 may be increased to separate constituents of the sample 152. For example, in one disclosure, after loading the sample the centrifuge 101 may accelerate the cartridge 150 to a speed of 1000 rpm at an acceleration of 2000 rpm/s. Upon reaching 1000 rpm the centrifuge 101 may further accelerate the cartridge 150 at 5000 rpm/s to a rate of 7000 rpm and hold at that rate for 90 seconds to separate the constituents. In another disclosure the centrifuge 101 may skip the initial transfer rotation speed and proceed directly from 0 rpm to a separation speed of 10,000 rpm at an acceleration of 2000 rpm/s. The separation step may occur at rotational speeds from 1000 rpm to 20,000 rpm depending upon the sample being analyzed, the radius of the separation chamber from the center of rotation, and the strength of the cartridge 150 to resist fracture. The duration of the separation may be carried out in a range of 10 second to 5 minutes.

In some disclosures, the sample 152 may be whole blood and the continued rotation of the cartridge 150 may separate the red blood cells from the plasma, as depicted in FIG. 12. For example, in the separation area 160 of the illustrated disclosure, the inner separation chamber 161 may act as a plasma compartment and the outer separation chamber 162 may act as a red blood cell trap. In response to high-speed rotation of the cartridge 150, the more dense red blood cells are pushed radially outward, while the less dense blood plasma moves radially inward into the plasma compartment 161.

The angled inner edge 164 of the outer separation chamber 162 may aid in separating the constituents of the sample in a similar manner as it promoted removal of air from the outer separation chamber 162, as described above. As the centrifuge 101 spins the cartridge 150, the more dense constituents will move outward and the less dense constituents will move inward. Accordingly, similar to the flow path of air in the outer separation chamber 162 during the filling process, the light constituents of the sample will move inward and then follow the angled inner edge 164 of the outer separation chamber 162 until they reach the constricted neck 163 and pass through to the inner separation chamber 161.

In some disclosures, the controller 140 may be configured to capture an image of the separation area or a portion thereof using the processing quality control camera after the separation process. The controller 140 may further be configured to analyze the image to determine the fill level of denser constituents of the sample in the separation area 160. In some disclosures the controller 140 is configured to confirm that certain denser constituents of the sample have moved outward from a predetermined fill level. The controller may likewise be configured to continue the analysis in response to such a confirmation.

For example, where the sample is whole blood, the controller 140 may be configured to analyze the image to determine the fill level of red blood cells in the separation area. If the fill level of the red blood cells is outside of a predetermined radius, the controller 140 may be configured to continue the analysis. On the other hand, if the fill level of the red blood cells is inside of the predetermined radius, the controller 140 may be configured to send a control signal to the centrifuge 101 to continue spinning the cartridge in order to further separate the constituents of the blood sample. For example an image may be captured and analyzed at 90 seconds of separation time. If the level of red blood cells is inward of a threshold distance of, for example 22mm from the center of rotation, the controller 140 may be configured to send a control signal to spin for another 30 seconds before capturing an additional image and reevaluating the level of red blood cells. In some disclosures, the duration or speed of this additional control signal may be based on the identified fill level of the red blood cells. Alternatively, the controller 140 may be configured to discontinue the analysis. In some disclosures, the method is configured to transfer a portion of the sample that excludes the red blood cells. The inclusion of red blood cells will add hemoglobin to the plasma, which can impact the analysis. Accordingly, identifying the fill level of the red blood cells allows the quality of the blood plasma that is transferred for further analysis to be determined.

Likewise, in some disclosures the image of the separation area 160 after the separation process may be analyzed by the controller to determine the clarity of the blood plasma in the inner separation chamber. Further, the controller 140 may be configured to proceed with the analysis in response to confirming that the blood plasma meets a threshold clarity.

Further still, in some disclosures the controller 140 may be configured to analyze the image of the separated blood sample to determine a hematocrit level of the blood based on the radial distance of the red blood cell line and the time of spin. Those skilled in the art will readily appreciate that for a given chamber geometry, rotation rate and rotation time, blood of a lower hematocrit level will exhibit a separation line at a larger radius than blood with a higher hematocrit level. For a given cartridge geometry and spin parameters, different hematocrit levels can be run and evaluated to determine a calibration table that is stored in the controller 140. When a sample of unknown hematocrit is run the separation line, after a predefined spin time, can be compared to values stored in the controller to determine the hematocrit level of the sample being run. Further, the controller 140 may be configured to proceed with the analysis in response to confirming that the hematocrit level is below a predetermined threshold.

### Transfer of Supernatant

As shown in FIG. 13, a portion of the sample 152 may be removed from the separation area 160 through a siphon 167 extending from the separation area 160. The siphon 167 may be in the form of a microfluidic channel with a cross sectional area of 1mm² that leads to a second chamber, such as mixing chamber 175. The siphon 167 may include a first section 168 extending from the separation area 160, a peak 169, and a second section 170 that extends from the peak 169 to the mixing chamber 175. The first section 168 of the siphon 167 extends from a siphon inlet 171 away from the inner separation chamber 161 toward the peak 169 in a direction that has a radially inward component. Further, the second section 170 extends from the peak 169 to a siphon outlet 172 point that is further radially outward than the siphon inlet 171 of the siphon. For example, the siphon inlet 171 may be at a radial position of 21mm from the center of rotation, whereas the siphon peak may be at 16mm from the center of rotation and the siphon outlet 172 may be at a radial distance of 30mm from the center of rotation. Other radial distances may be chosen to suit the needs of the application as long as the siphon outlet 172 is at greater radial distance than the siphon inlet 171 and the peak 169 is at a radial distance of less than both the siphon inlet 171 and siphon outlet 171. Thus, the peak 169 is the radially inner-most point of the siphon 167 and the siphon outlet 172 is radially outward compared to the siphon inlet 171. Accordingly, because the rotation of the centrifuge generally drives the sample radially outward, once a portion of the sample passes over the peak 169, the siphon 167 will drive a portion of the sample from the inner separation chamber 161 to the mixing chamber 175.

In some disclosures, the siphon may be primed, i.e., a portion of the sample may be compelled past the peak to begin the siphoning action, through capillary action. In other words, capillary force may draw the sample into the first section 168 of the siphon 167 and over the peak 169 until the siphoning action draws further fluid from the inner separation chamber 161. The cross-sectional area of the siphon 167 may be smaller, for example about 0.1 mm² to about 0.3 mm², or about 0.2 mm², to facilitate capillary action. In other disclosures, the siphon 167 may be primed through the use of pumps that draw the sample into the siphon 167 until the sample passes the peak.

Further, in some disclosures the siphon may be primed by acceleration. For example, in one disclosure after the cartridge 150 completed the separation step at 7000 rpm it is slowed down by centrifuge 101 to 3000 rpm at 2000 rpm/s to prepare for the siphon step. While the cartridge 150 is spinning in the first circumferential direction, inertia will cause the sample to be impelled to continue moving in that direction. Accordingly, if the cartridge 150 is decelerated quickly from 3000 rpm to 0 rpm for example at 8000 rpm/s, inertia will cause the sample 152 to continue moving in the first circumferential direction and the sample will flow through the first section 168 of the siphon 167 due to its extension along the first circumferential direction and through the peak 169 which is radially outward of the fill level of the separation area 160. At this point the centrifuge 101 may reverse the direction of spin to -1000 rpm at an acceleration of 2000 rpm/s and hold that speed. Centrifugal force will cause the fluid in channel 170 to move radially outward toward the siphon outlet 172 which is radially outward of the siphon inlet 171. The separation area 160 will continue to drain until the fill level is radially outward (or "drops below") the connection where the first section 168 of the siphon 167 opens into the inner separation chamber 161. This method of priming and siphon is significantly faster than the capillary action and or pump-based priming and siphon as the entire process can occur in several seconds. In some disclosures, the peak 169 is radially inward of the overflow channel 165, which prevents sample from flowing through the siphon 167 while the separation area 160 is being filled. Other rotational speeds and accelerations can be used as long as the acceleration is enough to force the fluid over the siphon peak 168 and the cartridge 150 continues to spin pulling fluid out of the separation area 160.

As stated above, the first section 168 of the siphon 167 extends in the first circumferential direction and radially inward. Further, in some disclosures, the shape of the first section 168 of the siphon 167 is particularly shaped to promote priming of the siphon 167. For example, in some disclosures a portion of the first section 168 at the end that is connected to the inner separation chamber 161 is substantially parallel to the first circumferential direction, e.g., within 10 degrees of parallel. As the first section 168 extends toward the peak 169 it gradually curves inward. As stated above, upon deceleration of the cartridge 150 the sample is urged in the first circumferential direction. Accordingly, with the first portion of the first section 168 substantially aligned with the first circumferential direction, the sample flows into the siphon 167 with great momentum. As a result of this momentum, the sample is able to reach and flow past the peak 169, thereby priming the siphon 167.

In some disclosures, the position of the connection between the first section 168 of the siphon 167 and the inner separation chamber 161 is selected to transfer a metered amount of sample through the siphon 167. For example, in the depicted disclosure in FIG. 13, the siphon 167 will transfer a precise amount of the sample, for example 50 microliters, based on the distance between the opening of the overflow channel 165 and the opening of the first section 168 of the siphon 167 in the radial direction. As the sample is transferred through siphon 167, the fill level in the inner separation chamber 161 will fall (i.e., move radially outward) and be replaced by air from the inlet channel 159 or overflow channel 165. Once the interface between the sample and the air reaches the first section 168 of the siphon 167, no additional amount of the sample will be pulled from the inner separation chamber 161. Accordingly, the position where the first section 168 opens into the inner separation chamber 161 may be used to define a metered amount of sample that is transferred to downstream chambers.

The position of the opening of the first section 168 of the siphon 167 into the inner separation chamber 161 may also be selected to limit the transfer through the siphon 167 of only certain constituents of the sample. For example, in the disclosures where the sample is whole blood and the separation chambers 162, 161 are used to separate the red blood cells from the plasma, the opening of the first section 168 may be positioned radially inward from the separated red blood cells. An unintentional inclusion of red blood cells in the sample that is transferred to the mixing chamber can result in hemoglobin contamination during the mixing process. Accordingly, it is advantageous to place the opening of the first section 168 to avoid the inclusion of red blood cells in the sample that is transferred through the siphon 167. Therefore, where the outer separation chamber 162 is a red blood cell trap that is configured to receive the red blood cells after the separation process, the opening of the first section 168 may be positioned radially inward from the from the red blood cell trap and within the plasma container. Likewise, the volume of the outer separation chamber 162 may be selected based on typical red blood cell volumes, for example a hematocrit level of 52% to ensure that the volume of the red blood cell trap can accommodate the volume of red blood cells present in most whole blood samples.

In some disclosures the outer separation chamber 162 extends away from the constricted neck 163 in the first circumferential direction. Accordingly, as the cartridge 150 is decelerated and the less dense constituents of the sample are urged through the siphon 167, the more dense constituents are likewise urged toward the closed end of the outer separation chamber 162 and away from the constricted neck 163 and the siphon entrance 171. For example, in disclosures using whole blood, as the blood plasma that is above the constricted neck 163 is transferred through the siphon 167, the red blood cells are urged toward the closed end of the red blood cell trap formed by outer separation chamber 162.

As discussed a large deceleration may be used to prime the siphon. As the cartridge is decelerating the dense components in the outer separation chamber move towards the closed end and away from constricted neck 163. However, there may be a density gradient in the outer separation chamber where the fluid density is higher toward the more radially outward portions of the outer separation chamber 162. In this case there can be some backflow at the top of the outer separation chamber where the separated components at the top of the chamber move toward the constricted neck 163. If those components move far enough toward the neck 163 they may be carried up into the upper separation chamber 161 and siphoned out of the upper separation chamber 162 into the mix chamber 176. While this can be controlled by spinning longer to further pack the dense components or by decelerating at a lower rate it may be advantageous to add baffles 191 in the lower separation chamber as shown in FIG 14. The baffles 191 may extend substantially through the depth of the outer separation chamber 162 and be positioned to impede motion of the dense constituents in the outer separation chamber. To facilitate the removal of air during initial filling of the lower separation chamber and to facilitate separation in the lower separation chamber the baffles 191 may be spaced away from the outer walls of the outer separation chamber 162. The baffles can be round, oval, square or rectangular. Further there can be multiple rows of baffles at different radial distances to form a grid. Further, the rows can be offset in the circumferential direction.

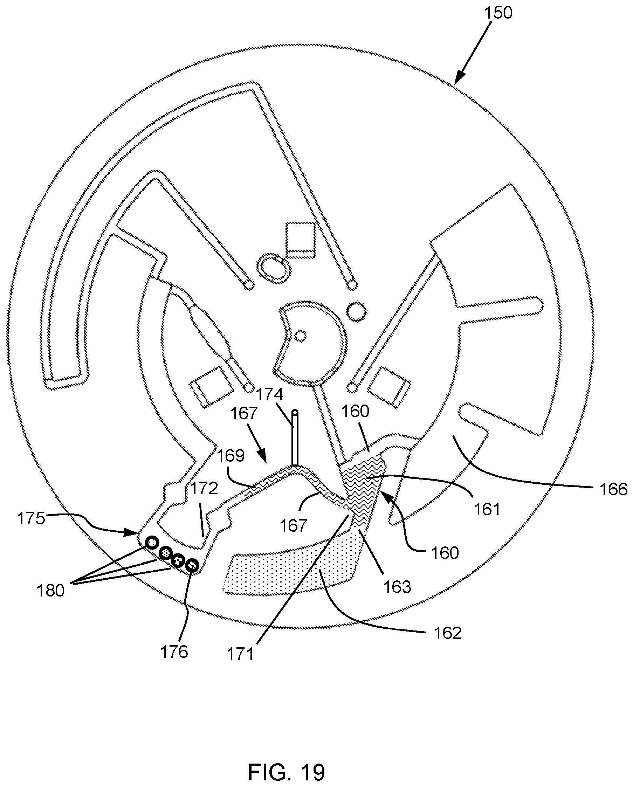

Figure 19 shows an alternative disclosure of siphon 167 including a vent channel 174 at the peak of the bend 169. The vent channel 174 extends from the peak of the bend 169 inward toward the center of the cartridge and is used to facilitate pump-based transfer of the sample from the separation area 160 to the mixing chamber 175. When the manifold 108 is not engaged the vent channel 174 is open to air. When manifold 108 registers on cartridge 150 the vent may be covered by a seal and closed. In an example method of operating the siphon 167 including the vent channel 174, after separation of the blood plasma the manifold 108 is brought into registration and contact with the cartridge 150. The draw pump 118 draws gas from the cartridge 150 through outlet port 156 pulling sample from separation area 160 through siphon line 167 and into mixing chamber 175. After a precise, predefined draw volume, the manifold 108 is raised and disconnected from the cartridge 150 and the vent channel 174 is opened to air. Centrifuge 101 then spins the cartridge 150 such that the sample remaining in siphon line 167 moves down both sides of the siphon line due to centrifugal force and away from the vent channel 174. The vent channel 174 enables movement of the sample by allowing air pulled in through the vent channel to displace the sample in the siphon line 167 as the sample moves away from the center of rotation toward mix chamber 175 and toward the separation area 160. Upon spinning of the cartridge, in the absence of the vent channel 174, siphon action would drain the separation area 160 up to the point where air reaches the entrance to the siphon line as previously discussed. In the vented disclosure of the siphon line 167 the amount of sample transferred to the mixing chamber can be determined by the pump draw volume rather than the geometry of the separation area and siphon line. Therefore, the volume of sample transferred is selectable rather than fixed.

### Sample Mixing

From the separation area 160, the blood plasma moves to the mixing chamber 175 which may have reagents therein. For example, the mixing chamber 175 may include lyophilized paramagnetic capture beads 177, a detection label, a control analyte, and a control label. Once in the mixing chamber 175, the blood plasma is mixed with the reagents by rapid acceleration and deceleration of the cartridge 150, all while continuing to rotate in the first circumferential direction, as shown in FIG. 14.

In some disclosures, the mixing of the blood plasma with the reagents is facilitated by a mixing ball 176 disposed in the mixing chamber 175. The acceleration and deceleration of the cartridge 150 as it rotates in the first circumferential direction causes the mixing ball 176 to move back-and-forth through the mixing chamber 175 bouncing off the walls thereof. For example, in one disclosure the centrifuge 101 may move the cartridge 150 at rotational speed between 200 rpm and 500 rpm accelerating and decelerating at 1500 rpm/s. This corresponds to a mix frequency of 5Hz. The turbulent movement of the mixing ball 176 initially rehydrates and releases the paramagnetic capture beads, detection label, control analyte, and control label into the plasma. The mixing ball 176 furthermore helps facilitate the binding kinetics of the target analyte to the paramagnetic capture beads 177 and detection label. After the mixing step, the target analyte and detection label may be attached together and to the paramagnetic capture beads that are dispersed throughout the blood plasma. In some disclosures, the rehydration of the reagents and the incubation of the target analyte occur in less than 20 minutes, for example, less than 10 minutes, or less than 5 minutes.

In some disclosures the mixing chamber 175 has geometric features that enhance the mixing ability of the mixing ball 176 by varying the direction of the mixing ball 176. For example, in some disclosures, the outer surface of the mixing chamber 175 includes a rough or textured surface to promote bouncing of the mixing ball as it rolls back and forth. Likewise, in some disclosures, the outer surface of the mixing chamber 175 may include a radially inward projection so as to cause the mixing ball to "jump" as it passes over the projection. Further, in some other disclosures the ends of the mixing chamber 175 are sloped in the radially inward direction to push the mixing ball inward at the ends of the mixing chamber and cause the mixing ball reverse directions and pass back through mixing chamber near the radially inner side of the mixing chamber. For example, both ends can have such a slope to enable a figure eight pattern of the mixing ball as the cartridge is moved rotational back and forth.

The term "mixing ball" is used herein in reference to the movement of this feature, and not with regard to any particular shape. Thus, the mixing ball 176 may be spherical in some disclosures, but have another shape in other disclosures. As examples, the mixing ball 176 may be oval, cubical, or star shaped. In some disclosures, the mixing ball is non-magnetic. The term non-magnetic, as used herein, includes those materials that are neither magnetic nor paramagnetic. Further, in some disclosures, the surface of the mixing ball includes a substance that has low reactivity. For example, in some the mixing ball 176 may include brass, glass or Teflon. Plastic, ceramic or other hard materials with a density higher than the sample may also be used for the mixing ball. In other disclosures, particularly those where paramagnetic capture beads are not used, the mixing ball 176 may include ferromagnetic materials, such as steel. Likewise, in some disclosures the mixing ball is coated with a substance that has a low reactivity.

The mixing chamber and surrounding channels include one or more features to retain the sample in the mixing chamber during a mixing process. In the cartridge 150 shown in FIGS. 9 to 19, both the ante mixing chamber channel, which is formed by the siphon 167, and the post mixing chamber channel 173 extend radially inward from the mixing chamber 175. Accordingly, centrifugal force as the cartridge 150 is spun by the centrifuge 101 urges the sample outward and into the mixing chamber 175.

Likewise, to prevent movement of the sample out of the mixing chamber by capillary action, at least one of the channels 167, 173 connected directly to the mixing chamber 175 may include a capillary break 178, 179. For example, in the cartridge 150 as shown in FIG. 14, both the ante mixing chamber channel 167 and the post mixing chamber channel 173 include respective capillary breaks 178, 179. Each of the capillary breaks 178, 179 is formed by a section of the respective channel 167, 173 that expands in the direction leading away from the mixing chamber 175. The expanding cross sectional area of the capillary break 178, 179 results in a reduced capillary force as the sample moves away from the mixing chamber 175. The use of capillary breaks 178, 179 reduces the effect of capillary action and keeps the incubated fluid in the chamber after the mixing and incubation of the paramagnetic capture beads, detection labels, control analytes, and control labels. This enables time for the magnet 130 to pull the paramagnetic beads out of suspension without the incubated fluid leaving the chamber 175 as is discussed in more detail below. In the disclosures shown in FIGS. 9-19, the capillary breaks are in the form of diamonds. In other disclosures, other shapes that expand as they project away from the mixing chamber 175 are also possible.

Further, the use of two capillary breaks may help balance the forces on the sample to retain the sample in the mixing chamber 175. For example, the mixing chamber 175 may be filled to such an extent that the fill line lies on both sides of the mixing chamber 175 within the capillary breaks 178, 179. Accordingly, if the sample moves toward one side of the mixing chamber, such that the fill line in one of the channels moves radially inward toward a widened section of the respective capillary break (e.g., 178), the capillary force within that channel will be reduced. Simultaneously, the fill line in the channel on the opposing side of the mixing chamber 175 should move radially outward and into a smaller cross-sectional area of the opposing capillary break (e.g., 179) where the capillary force will be stronger. Thus, the capillary forces on the sample from both capillary breaks will urge the sample to remain within the mixing chamber. To help facilitate this balancing effect, in some disclosures the two capillary breaks 178, 179 are at the same radial position.

The capillary breaks 178, 179 may also serve as a reservoir to hold a portion of the sample during the early stages of the mixing process. In some disclosures the reagents may be stored in a stable and dry form within the cartridge 150. For example, the reagents may be lyophilized prior to the analysis method of the disclosure. In such a case, the mixing of the blood plasma and the lyophilized reagents that occurs within the mixing chamber 175 may result in the release of air that was captured during the lyophilization process. Again, due to centrifugal force caused by rotation of the cartridge, this air will move radially inward and out of the sample as the mixing process ensues. Thus, the overall volume that is occupied by the sample when it first reaches the mixing chamber is larger than later in the mixing process when the air has been released. The capillary breaks 178, 179 can act as a reservoir to hold a portion of the sample until the air has been released and allowed to escape from the sample.

In some disclosures, the controller 140 may be configured to capture an image of the mixing chamber 175 or a portion thereof using the processing quality control camera after the transfer from the separation area 160. The controller 140 may further be configured to analyze the image to determine the fill level of the mixing chamber 175. Knowledge of the precise volume of the sample that is analyzed can be useful in determining an accurate concentration of the target analyte. Accordingly, the controller 140 may be configured to proceed with the analysis in response to determining that the volume in the mixing chamber 175 exceeds a threshold value. Furthermore, the controller 140 may be configured to use the volume of the sample that is analyzed for normalizing the data resulting from the analysis.

In some disclosures, the volume of the portion of the sample that is transferred to the mixing chamber 175 is larger than the volume of the mixing chamber, such that a portion of the sample remains in the ante mixing chamber channel 167 and the post mixing chamber channel 173. Thus, the controller 140 may be configured to identify the meniscus line of the sample in both channels from the image captured by the processing quality control camera and calculate the volume based on the position of these meniscus lines.

### Magnetic Movement of Sample

In some disclosures the analyzer 100 may include one or more magnets 130 (106) configured to move the paramagnetic capture beads as described in more detail below. As illustrated in the cross-sectional portion of the analyzer 100 shown in FIG. 20, each of which may be coupled to moveable stages 132, 133. The magnets 130, 131 may be positioned above or below the cartridge in order to enable movement of the paramagnetic capture beads 177 from outside of the cartridge 150. Linear movement of the magnet 130 in the radial direction and axial directions, combined with rotation of the cartridge 150 by the positioning motor 110 of the manifold allows the magnet 130 to be positioned over any portion of the cartridge 150 without the need to move the magnet 130 in the circumferential direction. Thus, in some disclosures, the stage 133 may be enabled to move the magnets 130, 131 forward and backward along the radial direction of the cartridge 150 using the radial magnet stage 133, as well as toward and away from the cartridge 150 in the axial direction to introduce or remove the magnetic attraction of the paramagnetic capture beads 177 using the axial magnet stage 132. In other, movable stages may be operable to move in three dimensions so as to move over any portion of the cartridge 150 without the need for the cartridge 150 to be rotated. In some disclosures, the magnet may be an electromagnet, while in other disclosures, the magnet may be a permanent magnet. Further, in some disclosures the electromagnets can be activated using AC or 126 current to further facilitate manipulation of the paramagnetic beads.

Once the contents of the mixing chamber 175 are thoroughly mixed and the target analytes are attached to the dispersed paramagnetic capture beads 177 (as shown in FIG. 14), the magnets 130, 131 may be introduced to move the paramagnetic capture beads 177 through the cartridge 150. With the magnet 130 placed adjacent to the mixing chamber 175, the cartridge 150 may be rotated back-and-forth over the magnet 130 in order to gather the paramagnetic capture beads 177 into a bolus, as shown in FIG. 16. In some disclosures, the controller 140 is configured to capture an image of the bead bolus after the paramagnetic capture beads have been collected using the magnet 130. Further, in some disclosures, the controller 140 is configured to measure the size of the paramagnetic bead bolus and proceed with the analysis if the size of the bead bolus is within a predetermined range. Otherwise the controller 140 may identify an error and discontinue the analysis.

In some disclosures, after the paramagnetic capture beads 177 are secured by the magnet 130, a wash buffer 182 may be pumped through the mixing chamber 175 so as to remove the blood plasma therefrom, as shown in FIG. 16. In some disclosures, during the purging of the blood plasma from the mixing chamber 175, the bolus of paramagnetic capture beads 177 may be held in a particular location of the mixing chamber 175 to avoid dispersion of the bolus. For example, the bolus may be positioned in a corner of the mixing chamber 175 during the purging of the blood plasma.

In some disclosures, the wash buffer 182 is delivered to the mixing chamber 175 via a wash chamber 181. The mixing chamber 175 and wash chamber 181 may be radially offset from one another. Further, in some disclosures, the microfluidic channel between the mixing chamber 175 and the wash chamber 181 may extend along a radial line and have a cross sectional area of 1mm². In other words, the microfluidic channel between the mixing chamber 175 and the wash chamber 181 does not extend along the circumferential direction. Accordingly, the fluid in the mixing chamber is not compelled to flow through the channel from the mixing chamber 175 to the wash chamber 181 during either acceleration or deceleration of the cartridge 150 during the mixing step. Moreover, as explained above, the post mixing chamber channel 173 may include a capillary break 178 that helps retain the sample in the mixing chamber 175.

In some disclosures, the wash buffer 182 is introduced into the cartridge 150 via the manifold 108 using a wash pump 116 and the elution buffer 185 may be pumped into the cartridge 150 via the manifold 108 using an elution pump 117, as shown in FIG. 8. In one disclosure, 150 microliters of wash buffer 182 may be pumped by the wash pump 116 through the wash supply port 111 of the manifold 108 and into the cartridge 150 through the wash inlet port 154 to fill the wash chamber. Likewise, in another disclosures, for example 25 microliters of elution buffer 185 may be pumped by the elution pump 117 through the elution supply port 112 of the manifold 108 into the cartridge 150 through the elution inlet port 155 to fill the elution chamber. As explained above, each of the supply ports 111, 112 can be carefully positioned to engage with the respective inlet ports 154, 155 so as to form a sealed connection.

In some disclosures, the controller 140 may be configured to capture an image of at least a portion of the wash chamber 181 after it is filled with wash buffer 182. Further, the controller 140 may be configured to analyze the image of the wash chamber 181 to confirm the absence of air within the wash chamber 181 or to confirm that the volume of any air bubbles within the wash chamber 181 is below a predetermined threshold. For example, the controller 140 may be configured to calculate the shape of any air bubbles within the wash chamber 181 and calculate the overall volume of air within the wash chamber 181. If the calculated volume of air is above a predetermined threshold, the controller may be configured to pump in more fluid or discontinue the analysis. Likewise, the controller may be configured to continue the analysis if the calculated volume of air is below a predetermined threshold or is zero. A similar process can be used with regard to air in the elution chamber 184. Specifically, the controller 140 may be configured to analyze the image of the elution chamber 184 to confirm the absence of air in the elution chamber 184 or to confirm that the volume of any air bubbles within the elution chamber 184 is below a predetermined threshold. For example, the controller 140 may be configured to calculate the shape of any air bubbles within the elution chamber 184 and calculate the overall volume of air within the elution chamber 184. If the calculated volume of air is above a predetermined threshold, the controller may be configured to discontinue the analysis. Likewise, the controller may be configured to continue the analysis if the calculated volume of air in the elution chamber 184 is below a predetermined threshold or is zero.

The analyzer 100 may also include a draw pump 118 that is coupled to the cartridge 150 via the manifold 108. In particular, the cartridge 150 may include a wash outlet port 156 and an elution outlet port 157 that are connected to the draw pump 118 via the manifold 108. In particular, the wash outlet port 156 may be coupled to the wash draw port 113 of the manifold 108 and the elution outlet port 157 may be coupled to the elution draw port 114 of the manifold 108. The inlet and outlet ports may form two respective fluid lines through the cartridge 150. In particular, wash inlet port 154 and wash outlet port 156 may form a wash line 183. Likewise, elution inlet port 155 and elution outlet port 157 may form an elution line 186 through the cartridge 150. Operation of the wash pump 116 and draw pump 118 may control the flow of wash buffer 182 through the wash line 183, while operation of the elution pump 117 and draw pump 118 may control the flow of elution buffer 185 through the elution line 186. Notably, in some disclosures, no wash buffer 182 or elution buffer 185 is actually drawn through the respective wash outlet port 156 and elution outlet port 157, but instead only gas is removed through these outlet ports as a way to control the movement of the respective fluids through the wash line 183 and elution line 186. For instance, waste chambers in the cartridge may be large enough that it is not necessary to remove fluid from the cartridge. Further, in some disclosures, each of the wash line 183 and elution line 186 may be coupled to a respective draw pump, rather than both being coupled to a single draw pump. Further, a single draw pump may be connected to the wash line 183 at one point in time and alternatively connected only to the elution line 186 at a different point in time.

In some disclosures, the wash pump 116 and the draw pump 118 are carefully controlled to avoid wash buffer 182 from entering the detection chamber 184. Further, in some disclosures, the analyzer operates the wash pump 116 and draw pump 118 to maintain a body of air in the connecting passage 187 as the wash fluid is drawn into the wash chamber, as shown in FIG. 16. For example, in some disclosures the controller operates the wash pump 116 and draw pump 118 at similar flow rates to transfer wash buffer along the wash line to avoid wash fluid from straying outside of the wash line. Similarly, in some disclosures the elution pump 117 and the draw pump 118 are controlled to avoid elution buffer 185 from entering the wash chamber 181.

Moreover, in some disclosures the elution buffer and wash buffer are introduced to the cartridge simultaneously and controlled so as to avoid cross contamination. For example, in some disclosures, as the wash buffer 182 and elution buffer 185 are introduced into the respective wash line 183 and elution line 186, the wash pump 116, the elution pump 117 and the draw pump 118 are controlled so as to form an air bubble in the connecting passage 187 connecting the wash line 183 and elution line 186. In particular, in some disclosures, this connecting passage 187 extends between the wash chamber 181 and the detection chamber 184. The air bubble forms a dam that prevents mixing of the wash buffer 182 and elution buffer 185, serving as an "air spring." Moreover, the air bubble can be visibly monitored to verify that the fluids are not mixing, as explained in more detail below. In some disclosures, this air bubble is maintained until the target analyte is moved into the detection chamber 184.

The use of a body of air, or air bubble, in the connecting passage avoids the need for a valve that controls flow between the wash chamber and elution chamber. In some disclosures, the controller 140 may be configured to capture an image of connecting passage 187 between the wash chamber 181 and the elution chamber 184 in order to confirm the presence of a body of air therein. If, after analyzing the image of the connecting passage 187, the controller identifies the presence of a body of air in the connecting passage 187, the controller 140 may be configured to proceed with the analysis. On the other hand, if the controller 140 does not identify a body of air in the connecting passage 187, the controller maybe configured to discontinue the analysis of the disclosure.

In some disclosures, at least one of the wash line or elution line includes an air trap. For example, in some disclosures the depth of the wash line 183 is increased in an area between the wash inlet port 154 and the wash chamber. This increase in the depth of the wash line 183 provides a space for any air that is pumped into the wash line to be caught. For example, in some disclosures, the analyzer holds the cartridge horizontally such that the depth direction is parallel to gravity. Accordingly, any air in the wash line 183 will float up and into the air trap caused by the increased depth of this section of the wash line. The elution line 186 may have a similar air trap in a vicinity of the elution inlet port 155.

With the paramagnetic capture beads 177 collected into a bolus in the mixing chamber 175, as shown in FIG. 15, the magnet 130 may be moved by the movable stage 133 in conjunction with rotation of the cartridge 150 to carry the bolus of paramagnetic capture beads 177 into the wash chamber 181. In some disclosures, the controller 140 may be configured to capture an image of at least a portion of the wash chamber 181 after the bead bolus of paramagnetic capture beads 171 has been transferred to the wash chamber 181. Further, in some disclosures, the controller 140 is configured to measure the size of the paramagnetic bead bolus in the wash chamber 181 and proceed with the analysis if the size of the bead bolus in the wash chamber 181 is within a predetermined range. Otherwise the controller 140 may identify an error and discontinue the analysis.

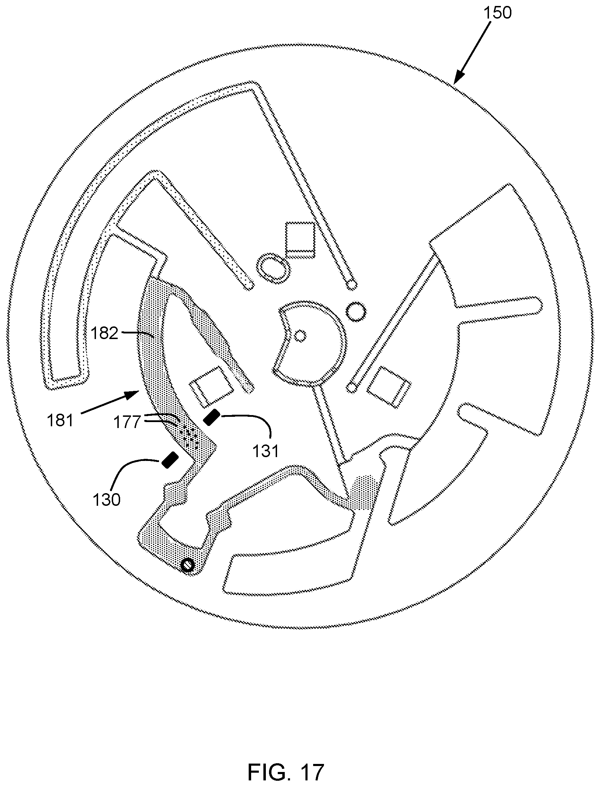

Once the paramagnetic capture beads 177 are disposed in the wash chamber 181, the cartridge 150 may be rotated back and forth to effectively wash the paramagnetic capture beads 177, removing all contaminants from the sample except the target analyte, detection label and any controls used in the system, as schematically shown in FIG. 17. In some disclosures, the spent wash buffer may be swept out of the wash chamber 181 and a new volume of wash buffer 182 added to the wash chamber 181 before repeating the wash step. The washing step may be performed several times, for example three or more times.

In some disclosures, a second magnet 131 may be introduced during the washing step to disperse and recondense the paramagnetic capture beads 177 during a series of steps of a washing operation. In particular, the magnet 130 and the second magnet 131 may be disposed on opposite sides of the wash chamber 181 in order to disperse and recondense the paramagnetic capture beads 177 as they move across the wash chamber 181. Spreading out the paramagnetic capture beads 177 allows them to be more efficiently washed by the wash buffer than if the beads held together in a bolus. Accordingly, the time and number of cycles needed for the washing step may be reduced compared to conventional washing methods.

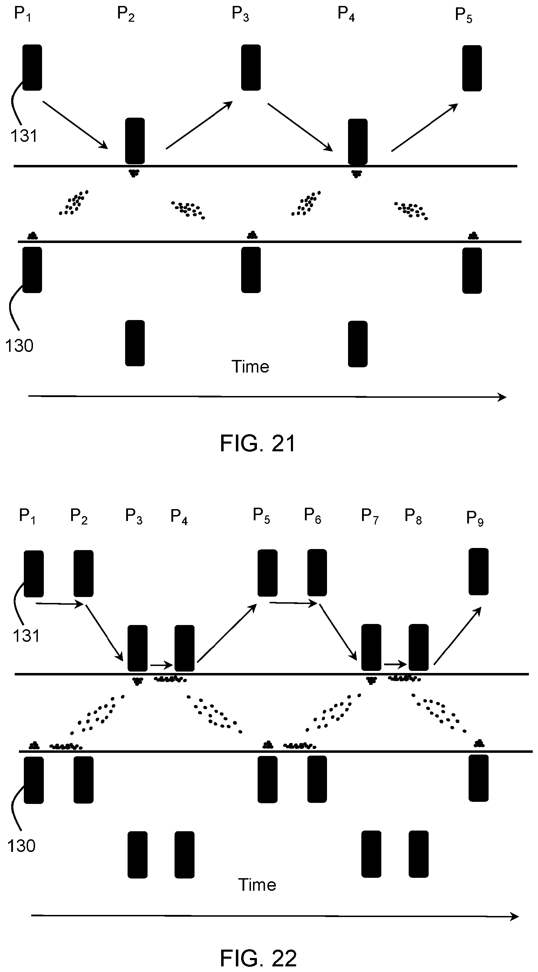

FIGS. 21 and 22 illustrate two example disclosures of a washing operation according to the present disclosure. FIG. 21 illustrates a washing operation in which two magnets 130, 131 are moved with respect to the wash chamber 181 in a saw tooth pattern. In particular, FIG. 21 illustrates five discrete locations P1-P5 that the first magnet 130 and second magnet 131 occupy during the saw tooth washing operation. In position P1, the second magnet 131 is remote from the wash chamber 181 while the first magnet 130 is adjacent to the wash chamber 181, which causes the paramagnetic capture beads to form a bolus adjacent to the first magnet 130. The magnets 130, 131 are then moved in the axial direction so that second magnet 131 draws near wash chamber 181 while first magnet 130 moves away from wash chamber 181. In concert with this movement, the cartridge 150 may also be rotated so that the magnets 130, 131 are also repositioned laterally with respect to the wash chamber 181. As the first magnet 130 moves away from the paramagnetic capture beads 177, the bolus is dispersed into the wash solution so that needless constituents of the blood plasma may be separated and washed from the paramagnetic capture beads 177. The dispersion of the paramagnetic capture beads 177 is illustrated in FIG. 21 between positions P1 and P2. As the second magnet 131 approaches the wash chamber 181, the paramagnetic capture beads 177 are drawn out of suspension and again into a tight bolus. The dispersion and recondensing steps can then be repeated in the opposite direction as the magnets 130, 131 move from position P2 to position P3. Likewise, this process can be continued in a sawtooth pattern for several additional steps.

FIG. 22 illustrates another disclosure of a washing operation in which the two magnets 130, 131 are moved with respect to the wash chamber 181 in a square wave pattern. In particular, FIG. 22 illustrates nine discrete locations P1-P9 that the first magnet 130 and second magnet 131 occupy during the saw tooth washing operation. Again, in position P1 the second magnet 131 is remote from the wash chamber 181 while the first magnet 130 is adjacent to the wash chamber 181, which causes the paramagnetic capture beads to form a bolus adjacent to the second magnet 131. The cartridge 150 is then rotated such that the magnets 130, 131 move with respect to the wash chamber 181. Advantageously, the cartridge 150 may be rotated at a sufficient velocity to spread the paramagnetic capture beads 177 along the surface of the wash chamber 181, thereby dispersing the paramagnetic capture beads along the surface of the chamber 181 in the wash solution. The magnets 130, 131 are then moved to position P3 such that the second magnet 131 draws near wash chamber 181 while first magnet 130 moves away from wash chamber 181. Again, as the first magnet 130 moves away from the paramagnetic capture beads 177, the bolus is dispersed into the wash solution so that needless constituents of the blood plasma may be separated and washed from the paramagnetic capture beads 177. Likewise, as the second magnet 131 approaches the wash chamber 181, as shown at position P3, the paramagnetic capture beads 177 are drawn out of suspension and against the wash chamber wall.

While the disclosures of wash operations shown in FIGS. 21 and 22 include recondensing the paramagnetic capture beads into a tight bolus, in other disclosures, the paramagnetic capture beads may be directed through the wash chamber without strictly being coalesced into a bolus during the operation. For example, during the steps of the operation the beads may remain relatively dispersed in the wash fluid but moved back and forth and along the length of the wash chamber by the magnets.

As stated above, in some disclosures, the magnet 130 and second magnet 131 are positioned on opposite sides of the cartridge 150, for example above and below the cartridge 150. In other disclosures, the magnets 130, 131 are disposed on the same side of the cartridge 150 but on opposite sides of the wash chamber 181 with respect to the radial direction. Further, in some disclosures, the magnets 130, 131 spread the paramagnetic capture beads 177 along the length of the wash chamber 181. Moreover, in some disclosures, the distance between the first magnet 130 and the second magnet 131 is varied using the movable stage 132 during the washing step. This relative movement of the magnets 130, 131 may promote the disruption of the bolus of paramagnetic capture beads 177, enhancing the washing operation.

After the washing operation, the paramagnetic capture beads 177 may be gathered again with the first magnet 130 and moved through the connecting passage 187 into the detection chamber 184, which is filled with elution buffer 185, as shown in FIG. 19. While holding the paramagnetic capture beads 177 using one or more magnets 130, 131, the cartridge 150 may be rotated back-and-forth to pass the paramagnetic capture beads 177 through the detection chamber 184 and elution buffer 185, which removes the bonds between the paramagnetic capture beads 177 and the target analyte and between the label and target analyte. This leaves a pure fluorochrome conjugate suspension in the elution buffer 185 within the detection chamber 184.

To enhance elution of the target analyte and labels, a magnetic elution operation may be used that is similar to the wash operations explained above. For example, the magnets 130, 131 may move with respect to the detection chamber 184 in a particular pattern, such as those shown in FIGS. 21 and 22. Controlling the paramagnetic beads in a controlled manner similar to that of the washing operation enhances the magnetic elution operation.

After the elution process has been carried out, the paramagnetic capture beads 177 may be moved outside of the detection chamber 184, or to one end of the detection chamber 184 so as to avoid interfering with the optical system 120. The optical system 120 of the analyzer 100 may then be activated to analyze the solution in the detection chamber 184 so as to determine the presence or concentration of target analyte in the volume of fluid in the detection chamber 184, as explained above.

As described herein, the optical system 120 of the analyzer 100 includes a second electromagnetic radiation source 128 and a second detector 129 for a multiplexing operation. In some disclosures, the analyzer second electromagnetic radiation source 128 and second detector 129 may be used for determining the presence of a second target analyte in the sample. In other disclosures, the second electromagnetic radiation source 128 and the second detector 129 may be used to measure a concentration of a control analyte in the cartridge 150. For example, the cartridge 150 may include a precise and known quantity of the control analyte. Accordingly, the measured concentration of the control analyte may be used as a comparator for the target analyte. This measured concentration can then be used to adapt the detected concentration of the target analyte.

For example, if the measured concentration of the control analyte is only 95% of the actual known concentration of the control analyte, the controller 140 can use this percentage difference to adapt the detected concentration of the target analyte. For example, the controller 140 may determine that the analyzer 100 is also only detecting 95% of the target analyte in the sample, and adjust the calculated concentration accordingly.

In some disclosures, the electromagnetic radiation from the first electromagnetic radiation source 121 and the second electromagnetic radiation source 128 are directed to the cartridge using the same objective. Indeed, in some disclosures, the electromagnetic radiation from the two sources is directed to the same interrogation space. In some disclosures, the first electromagnetic radiation source 121 and the second electromagnetic radiation source 128 emit electromagnetic radiation of different wavelengths, for example, different colors.

The disclosure provides systems and methods for highly sensitive detection and quantitation of one or more target analytes, such as markers for biological states.

### Singleplex and Multiplex Assays

Described herein are systems and methods that can perform a "singleplex" assay of a sample to detect and analyze a single type of target analyte in the sample or systems and methods that can perform a "multiplex" assay of a sample to detect and analyze multiple (e.g., two, three or more) different types of target analytes in the sample. Using the multiplexed systems and methods described herein may provide for more rapid detection and analysis of multiple target analytes, using reduced sample volume, and reduced reagent volume than may be required to perform a similar analysis of those target analytes via singleplex assays. Further, the multiplexed systems and methods described herein can allow analysis of a sample including a target analyte to be compared to a control assay of a known concentration.

To detect and analyze multiple, different types of target analytes in a sample, the multiplexed analyzer system can distinguish one type of target analyte from the others. This can be achieved, in part, by labeling the different target analytes with different labels, which have excitation wavelength bands and/or emission wavelength bands that differ from one another. In some implementations, the different labels have excitation wavelength bands and/or emission wavelength bands with relatively little overlap or no overlap. In other implementations, there may be some overlap among the excitation wavelength bands and/or the emission wavelength bands of the labels. Multiplexing can also be achieved by implementing more than one fluidic circuit on the same cartridge with each fluidic circuit spatially distinct and carrying reagents for different target analytes. With different fluidic circuits it is not necessary for the different target labels to have different excitation and emission wavelengths. The additional circuits may collect sample from the same sample chamber or from different sample chambers.

### Electromagnetic Radiation Power and Bin Size

In the optical system, the electromagnetic radiation source 121 may be set so that the wavelength of the electromagnetic radiation is sufficient to excite a fluorescent label attached to the target analyte. In some disclosures, the electromagnetic radiation source 121 is a laser that emits light in the visible spectrum. In some disclosures, the laser is a continuous wave laser with a wavelength of 639 nm, 532 nm, 488 nm, 422 nm, or 405 nm. Any continuous wave laser with a wavelength suitable for exciting a fluorescent moiety as used in the methods and compositions of the disclosure can be used without departing from the scope of the disclosure. The power setting for the laser is generally between 1mW and 100mW. However, those skilled in the art will appreciate the laser power can be any setting to achieve the optimal signal to noise ratio of the measurement. To do so the laser power should be set to achieve as many excitation emission cycles as possible during the dwell time of the label in the interrogation space. The detector bin time should also be set accordingly. A bin time that is longer than the time it takes to photo bleach the label and or longer than the dwell time of the label in the interrogation space will simply enable the collection of excess noise. A laser power setting that is too low or too high or a bin time setting that is to long will not yield the highest possible signal to noise ratio.

As the interrogation space in the analyzer 100 passes over the labeled target analyte, photons emitted by the fluorescent particles are registered by the detector 122 with a time delay indicative of the time for the interrogation space to pass over the labeled particle. The photon intensity is recorded by the detector 122 and the sampling time is divided into bins, wherein the bins are uniform, arbitrary time segments with freely selectable time channel widths. The number of signals contained in each bin is evaluated. One or more of several statistical analytical methods are used to determine when a label or particle is present or when a section of bins contains an artifact. Sections of bins containing artifacts are discarded while single bins or sections of bins containing a label are counted. The number of labels counted is indicative of the number of target analytes present in the sample.

### Interrogation Volume

An interrogation volume can be thought of as an effective volume of sample in which a target analyte of interest can be detected when present. Although there are various ways to calculate the interrogation volume of the sample, the simplest method for determining the effective volume (V) of the interrogation volume is to calculate the effective cross section of the detection volume. Because the detection volume is typically swept through the sample by translating the detection volume through the stationary sample, the volume is typically the result of the cross sectional area of the detection volume being swept through some distance during the time of measurement. As previously discussed the lateral extent of the cross sectional area of interrogation volume (perpendicular to the direction of motion of the laser relative to the sample and perpendicular to the direction of propagation of the laser light) is limited by the numerical aperture at which the laser source is imaged in the sample space. The longitudinal size of the interrogation volume (along the direction of propagation of the laser) is determined by the size of the confocal stop chosen. If the sample concentration (C) is known and the number of molecules detected (N) during a period of time is known, then the sample volume consists of the number of molecules detected divided by the concentration of the sample, or V=N/C (where the sample concentration has units of molecules per unit volume).

For example, in some disclosures of the system described herein, all photons detected are counted and added up in 100 microsecond segments (photon counting bins). If a molecule of interest is present in the 100 microsecond segment, the count of photons detected is typically significantly higher than background. Therefore, the distance the detection volume has moved with respect to the sample is the appropriate distance to use to calculate the volume sampled in a single segment, i.e., the interrogation volume. In this example, if the sample is analyzed for 60 seconds, then effectively 600,000 segments are scanned. If the effective volume is divided by the number of segments, the resulting volume is in essence the volume of a single segment, i.e., the interrogation volume. Mathematically, the volume of the single segment, i.e., the interrogation volume (Vs), equals the number of molecules detected (N) divided by the concentration of the sample multiplied by the number of segment bins (C·n-where n represents the number of segment bins during the time the N number of molecules were counted). For exemplary purposes only, consider that a known standard of one femtomolar concentration is run through 600,000 segments, and 20 molecules of the standard are detected. Accordingly, the interrogation volume, Vs, equals N/(C·n) or 20/(602.214·6E5), or 55.351 µm3. Thus, in this example, the interrogation space volume, which is the effective volume for one sample corresponding to one photon counting bin, is 55.351 µm3.

### Detectors

In some disclosures, light emitted by a fluorescent label after exposure to electromagnetic radiation is detected. The emitted light can be, e.g., ultra-violet, visible or infrared. For example, the first detector 122 may capture the amplitude and duration of photon bursts from a fluorescent moiety, and convert the amplitude and duration of the photon bursts to electrical signals. Detection devices such as CCD cameras, video input module cameras, and Streak cameras can be used to produce images with contiguous signals. Other disclosures use devices such as a bolometer, a photodiode, a photodiode array, avalanche photodiodes, and photomultipliers which produce sequential signals. Any combination of the aforementioned detectors can be used.

### Molecules for Concentration Analysis

The instruments, kits and methods of the disclosure can be used for the sensitive detection and determination of concentration of a number of different types of target analytes, such as markers of biological states.

Examples of molecules or "analytes" that can be detected using the analyzer and related methods of the disclosure include: biopolymers such as proteins, nucleic acids, carbohydrates, and small molecules, both organic and inorganic. In particular, the instruments, kits, and methods described herein are useful in the detection of target analytes of proteins and small molecules in biological samples, and the determination of concentration of such molecules in the sample.

The molecules detected by the present systems and methods can be free or can be part of a complex, e.g., an antibody-antigen complex, or more generally a protein-protein complex, e.g., complexes of troponin or complexes of prostate specific antigen (PSA).

Described herein, the disclosure provides compositions and methods for the sensitive detection of biological markers, and for the use of such markers in diagnosis, prognosis, and/or determination of methods of treatment.

Markers can be, for example, any composition and/or molecule or a complex of compositions and/or molecules that is associated with a biological state of an organism (e.g., a condition such as a disease or a non-disease state). A marker can be, for example, a small molecule, a polypeptide, a nucleic acid, such as DNA and RNA, a lipid, such as a phospholipid or a micelle, a cellular component such as a mitochondrion or chloroplast, etc. Markers contemplated by the disclosure can be previously known or unknown. For example, in some disclosures, the methods herein can identify novel polypeptides that can be used as markers for a biological state of interest or condition of interest, while in other disclosures, known polypeptides are identified as markers for a biological state of interest or condition. Using the systems of the disclosure it is possible that one can observe those markers, e.g., polypeptides with high potential use in determining the biological state of an organism, but that are only present at low concentrations, such as those "leaked" from diseased tissue. Other high potentially useful markers or polypeptides can be those that are related to the disease, for instance, those that are generated in the tumor-host environment. Any suitable marker that provides information regarding a biological state can be used in the methods and compositions of the disclosure. A "marker," as that term is used herein, encompasses any molecule that can be detected in a sample from an organism and whose detection or quantitation provides information about the biological state of the organism.

Biological states include but are not limited to phenotypic states; conditions affecting an organism; states of development; age; health; pathology; disease detection, process, or staging; infection; toxicity; or response to chemical, environmental, or drug factors (such as drug response phenotyping, drug toxicity phenotyping, or drug effectiveness phenotyping).

The term "organism" as used herein refers to any living being comprised of a least one cell. An organism can be as simple as a one cell organism or as complex as a mammal. An organism of the disclosure is preferably a mammal. Such mammal can be, for example, a human or an animal such as a primate (e.g., a monkey, chimpanzee, etc.), a domesticated animal (e.g., a dog, cat, horse, etc.), farm animal (e.g., goat, sheep, pig, cattle, etc.), or laboratory animal (e.g., mouse, rat, etc.). Preferably, an organism is a human.

### Labels

Described herein, the disclosure provides methods and compositions that include labels for the highly sensitive detection and quantitation of molecules, e.g., of markers.

Many strategies can be used for labeling target analytes to enable their detection or discrimination in a mixture of particles. The labels can be attached by any known means, including methods that utilize non-specific or specific interactions of label and target analyte. Labels can provide a detectable signal or affect the mobility of the particle in an electric field. Labeling can be accomplished directly or through binding partners.

In some disclosures, the label comprises a binding partner to the molecule of interest, where the binding partner is attached to a fluorescent moiety. The compositions and methods of the disclosure can use highly fluorescent moieties. Moieties suitable for the compositions and methods of the disclosure are described in more detail below. Fluorescent molecules may be attached to binding partners by any known means such as direct conjugation or indirectly (e.g., biotin/streptavidin).

The fluorescent moieties can be fluorescent dye molecules. Examples of fluorescent molecules include but are not limited to ALEXA FLUOR^{®} 488, ALEXA FLUOR^{®} 532, ALEXA FLUOR^{®} 647, ALEXA FLUOR^{®} 680 or ALEXA FLUOR^{®} 700 Brilliant Violet^{™} molecules (BD Biosciences) such as Brilliant Violet 421^{™}, Brilliant Violet 510^{™}, Brilliant Violet 570^{™},| Brilliant Violet 605and ATTO^{™} dyes (ATTO TECH GmbH) such as ATTO^{™} 532. In some disclosures, the dye molecules are ALEXA FLUOR^{®} 647 dye molecules.

### Binding Partners

In some disclosures, the binding partner comprises an antibody. In some disclosures, the antibody is a monoclonal antibody. In other disclosures, the antibody is a polyclonal antibody.

The antibody can be specific to any suitable marker. In some disclosures, the antibody is specific to a marker that is selected from the group consisting of cytokines, growth factors, oncology markers, markers of inflammation, endocrine markers, autoimmune markers, thyroid markers, cardiovascular markers, markers of diabetes, markers of infectious disease, neurological markers, respiratory markers, gastrointestinal markers, musculoskeletal markers, dermatological disorders, and metabolic markers.

Any suitable binding partner with the requisite specificity for the form of molecule, e.g., a marker, to be detected can be used. If the molecule, e.g., a marker, has several different forms, various specificities of binding partners are possible. Suitable binding partners are known in the art and include antibodies, aptamers, lectins, and receptors. A useful and versatile type of binding partner is an antibody.

Capture binding partners and detection binding partner pairs, e.g., capture and detection antibody pairs, can be used in disclosures of the disclosure. Thus, in some disclosures, a heterogeneous assay protocol is used in which, typically, two binding partners, e.g., two antibodies, are used. One binding partner is a capture partner, usually immobilized on a solid support, and the other binding partner is a detection binding partner, typically with a detectable label attached. Antibody pairs can be designed and prepared by methods well-known in the art. Compositions of the disclosure include antibody pairs wherein one member of the antibody pair is a label as described herein, and the other member is a capture antibody.

In some disclosures it is useful to use an antibody that cross-reacts with a variety of species, either as a capture antibody, a detection antibody, or both. Such disclosures include the measurement of drug toxicity by determining, e.g., release of cardiac troponin into the blood as a marker of cardiac damage. A cross-reacting antibody allows studies of toxicity to be done in one species, e.g. a non-human species, and direct transfer of the results to studies or clinical observations of another species, e.g., humans, using the same antibody or antibody pair in the reagents of the assays, thus decreasing variability between assays. Thus, in some disclosures, one or more of the antibodies for use as a binding partner to the marker of the molecule of interest, e.g., cardiac troponin, such as cardiac troponin I, can be a cross-reacting antibody. In some disclosures, the antibody cross-reacts with the marker, e.g. cardiac troponin, from at least two species selected from the group consisting of human, monkey, dog, and mouse. In some disclosures, the antibody cross-reacts with the marker, e.g., cardiac troponin, from the entire group consisting of human, monkey, dog, and mouse.

The above detailed description describes various features and functions of the disclosed systems, devices, and methods with reference to the accompanying Figures. In the Figures, similar symbols typically identify similar components, unless context dictates otherwise. The illustrative disclosures described in the detailed description, Figures, and claims are not meant to be limiting. Other disclosures can be utilized, and other changes can be made, without departing from the scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

## Claims

1. A method of mixing a liquid in a cartridge (150) in the form of a flat disc, the method comprising:
introducing a liquid to a mixing chamber (175) of the cartridge (150) through a channel that extends radially inward from the mixing chamber, the mixing chamber including a mixing ball (176) therein;
rotating the cartridge (150) in a first circumferential direction so as to urge the liquid radially outward and retain the liquid in the mixing chamber (175); **characterized in that**,
while continuing to rotate the cartridge (150) in the first circumferential direction, intermittently accelerating and decelerating the rotation of the cartridge (150) so as to move the mixing ball (176) back-and-forth recursively through the mixing chamber (150).

2. The method according to claim 1, wherein the mixing chamber (175) is provided with lyophilized reagents prior to the introduction of the liquid, and
wherein mixing resulting from the movement of the mixing ball (176) through the mixing chamber (175) releases gas from the lyophilized reagents into the liquid.

3. The method according to claim 2, wherein the released gas moves radially inward and out of the mixing chamber (175).

4. The method according to claim 1, wherein the mixing ball (176) is nonmagnetic.

5. The method according to claim 1, wherein a rotational speed of the cartridge (150) is maintained in a range of 200 to 500 rpm while intermittently accelerating and decelerating, corresponding to a mix frequency of 5Hz.

6. The method according to claim 1, wherein the mixing chamber (175) is provided with paramagnetic capture beads (177) prior to introduction of the liquid.

7. The method according to claim 1, wherein the channel extending radially inward from the mixing chamber (175) includes an expanded cross-sectional area that forms a capillary break (178).

8. The method according to claim 1, wherein the cartridge (150) includes a second channel extending radially inward from the mixing chamber (175), coupling the mixing chamber with a wash chamber (181).

## Patentansprüche

1. Verfahren zum Mischen einer Flüssigkeit in einer Kartusche (150) in Form einer flachen Scheibe, wobei das Verfahren Folgendes umfasst:
Einbringen einer Flüssigkeit in eine Mischkammer (175) der Kartusche (150) über einen Kanal, der sich von der Mischkammer aus radial nach innen erstreckt, wobei die Mischkammer darin eine Mischkugel (176) umfasst;
Drehen der Kartusche (150) in eine erste Umfangsrichtung, um die Flüssigkeit radial nach außen zu zwingen und die Flüssigkeit in der Mischkammer (175) zurückzuhalten;
**dadurch gekennzeichnet, dass**
während des anhaltenden Drehens der Kartusche (150) in die erste Umfangsrichtung das Drehen der Kartusche (150) intermittierend beschleunigt und verlangsamt wird, um die Mischkugel (176) rekursiv vorwärts und rückwärts durch die Mischkammer (150) zu bewegen.

2. Verfahren nach Anspruch 1, wobei die Mischkammer (175) vor dem Einbringen der Flüssigkeit mit lyophilisierten Reagenzien bereitgestellt wird und
wobei das Mischen, das aus der Bewegung des Mischballs (176) durch die Mischkammer (175) resultiert, Gas aus den lyophilisierten Reagenzien in die Flüssigkeit freisetzt.

3. Verfahren nach Anspruch 2, wobei das freigesetzte Gas sich radial in die Mischkammer (175) hinein und daraus heraus bewegt.

4. Verfahren nach Anspruch 1, wobei die Mischkugel (176) nichtmagnetisch ist.

5. Verfahren nach Anspruch 1, wobei eine Drehgeschwindigkeit der Kartusche (150) in einem Bereich von 200 bis 500 U/min gehalten wird, während intermittierend beschleunigt und verlangsamt wird, was einer Mischfrequenz von 5 Hz entspricht.

6. Verfahren nach Anspruch 1, wobei die Mischkammer (175) vor dem Einbringen der Flüssigkeit mit paramagnetischen Einfangkügelchen (177) bereitgestellt wird.

7. Verfahren nach Anspruch 1, wobei der Kanal, der sich von der Mischkammer (175) aus radial nach innen erstreckt, einen erweiterten Querschnittsbereich umfasst, der einen Kapillarbruch (178) ausbildet.

8. Verfahren nach Anspruch 1, wobei die Kartusche (150) einen zweiten Kanal umfasst, der sich von der Mischkammer (175) aus radial nach innen erstreckt, welcher die Mischkammer mit einer Waschkammer (181) verbindet.

## Revendications

1. Procédé de mélange d'un liquide dans une cartouche (150) sous la forme d'un disque plat, le procédé comprenant les étapes consistant à :
introduire un liquide dans une chambre de mélange (175) de la cartouche (150) à travers un canal qui s'étend radialement vers l'intérieur à partir de la chambre de mélange, la chambre de mélange comprenant une bille de mélange (176) en son sein ;
faire tourner la cartouche (150) dans une première direction circonférentielle de manière à pousser le liquide radialement vers l'extérieur et à retenir le liquide dans la chambre de mélange (175) ; **caractérisé en ce que**
tout en continuant à faire tourner la cartouche (150) dans la première direction circonférentielle, accélérer et décélérer de manière intermittente la rotation de la cartouche (150) de manière à déplacer la bille de mélange (176) de manière récurrente en va-et-vient à travers la chambre de mélange (150).

2. Procédé selon la revendication 1, dans lequel la chambre de mélange (175) est pourvue de réactifs lyophilisés avant l'introduction du liquide, et
dans lequel le mélange résultant du déplacement de la bille de mélange (176) à travers la chambre de mélange (175) libère du gaz à partir des réactifs lyophilisés jusque dans le liquide.

3. Procédé selon la revendication 2, dans lequel le gaz libéré se déplace radialement vers l'intérieur et vers l'extérieur de la chambre de mélange (175).

4. Procédé selon la revendication 1, dans lequel la bille de mélange (176) est non magnétique.

5. Procédé selon la revendication 1, dans lequel une vitesse de rotation de la cartouche (150) est maintenue dans une plage de 200 à 500 tr/min tout en accélérant et en décélérant par intermittence, correspondant à une fréquence de mélange de 5 Hz.

6. Procédé selon la revendication 1, dans lequel la chambre de mélange (175) est munie de perles de capture paramagnétiques (177) avant l'introduction du liquide.

7. Procédé selon la revendication 1, dans lequel le canal s'étendant radialement vers l'intérieur à partir de la chambre de mélange (175) comprend une surface de section transversale élargie qui forme une rupture capillaire (178).

8. Procédé selon la revendication 1, dans lequel la cartouche (150) comprend un second canal s'étendant radialement vers l'intérieur à partir de la chambre de mélange (175), couplant la chambre de mélange à une chambre de lavage (181).
